(19) **Europäisches Patentamt European Patent Office Office européen des brevets**

(11) **EP 2 475 975 B1**

(12) **FASCICULE DE BREVET EUROPEEN**

(45) Date de publication et mention de la délivrance du brevet:
**12.12.2018 Bulletin 2018/50**

(51) Int Cl.:
**G01N 21/47** *(2006.01)*　　**G01N 21/31** *(2006.01)*
**G01N 33/02** *(2006.01)*

(21) Numéro de dépôt: **10762710.1**

(22) Date de dépôt: **03.09.2010**

(86) Numéro de dépôt international:
**PCT/FR2010/000599**

(87) Numéro de publication internationale:
**WO 2011/027052 (10.03.2011 Gazette 2011/10)**

(54) **SPECTROMETRE OPTIQUE AUTONOME ET PORTABLE**

AUTONOMES UND TRAGBARES OPTISCHES SPEKTROMETER

SELF-CONTAINED AND PORTABLE OPTICAL SPECTROMETER

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(30) Priorité: **07.09.2009 FR 0904241**

(43) Date de publication de la demande:
**18.07.2012 Bulletin 2012/29**

(73) Titulaire: **IRSTEA**
**92761 Antony Cedex (FR)**

(72) Inventeurs:
- **PELLENC, Roger**
  **F-84120 Pertuis (FR)**
- **GIALIS, Jean-Marc**
  **F-84460 Cheval Blanc (FR)**
- **FERRANDIS, Jean-Louis**
  **F-84240 La Motte d'Aigues (FR)**
- **BOURELY, Antoine**
  **F-84240 La Tour d'Aigues (FR)**
- **BELLON MAUREL, Véronique**
  **F-34790 Grabels (FR)**
- **ROGER, Jean-Michet**
  **F-34000 Montpellier (FR)**

(74) Mandataire: **Weber, Etienne Nicolas et al**
**Cabinet Marek**
**28, rue de la Loge**
**Boîte Postale 42413**
**13201 Marseille Cedex 02 (FR)**

(56) Documents cités:
**WO-A1-98/38494　　WO-A2-02/088678**
**US-A1- 2004 130 720　　US-A1- 2006 118 726**

- **LARRAÍN M, ET AL: "A multipurpose portable instrument for determining ripeness in wine grapes using NIR spectroscopy", IEEE TRANSACTIONS ON INSTRUMENTATION AND MEASUREMENT, vol. 57, no. 2, 2008, pages 294-302, XP002592089,**
- **GERAUDIE V, ET AL: "A revolutionary device for predicting grape maturity based on NIR spectrometry", FRUCTIC 09, 8TH FRUIT NUT AND VEGETABLE PRODUCTION ENGINEERING SYMPOSIUM, 5 septembre 2009 (2009-09-05), pages 1-8, XP002592090, Conception: Chile**
- **BLAKEY, ET AL: "The potential of near-infrared spectroscopy in the avocado industry", SOUTH AFRICAN AVOCADO GROWERS' ASSOCIATION YEARBOOK, vol. 31, 2008, pages 47-50, XP002592091,**
- **DU PLESSIS, C S: "Optimum maturity and quality parameters in grapes: a review", S. AFR. J. ENOL. VITIC., vol. 5, no. 1, 1984, pages 35-42, XP002592092,**

**Description**

La présente invention concerne un spectromètre optique, en particulier spectromètre optique autonome et portable, destiné à analyser le spectre lumineux rétro diffusé par un échantillon soumis à un éclairage, afin de déterminer une teneur d'au moins un composé constituant l'échantillon.

Dans le domaine de l'analyse de la teneur d'un composé d'un échantillon de matière, il est connu de réaliser une détermination au moyen d'un spectromètre de masse, au moyen d'un spectromètre optique ou encore par chromatographie. Ces principes de mesure nécessitent des appareils de laboratoire qui sont des équipements très généralistes, lourds et coûteux.

Le document WO 98/38494 décrit un dispositif et un procédé de spectrométrie utilisant le mode de réflexion de surface qui peut être spéculaire ou diffuse en fonction de l'état de la surface de l'échantillon analysé. Ce mode de réflexion de surface ne peut être appliqué qu'à des analyses de la surface externe d'un échantillon opaque tel qu'un échantillon de viande.

Il n'est pas utilisable pour la détection de la présence d'un ou plusieurs composés dans un échantillon au moins partiellement translucide tel qu'un grain de raisin ou autres fruits, par exemple pour déterminer le degré de mûrissement de ces derniers, nécessitant une pénétration de la lumière à l'intérieur dudit échantillon.

Le document US2004/130720 décrit un dispositif autonome et portatif pour la mesure optique de la qualité de fruits. Il intègre une source d'éclairage et des capteurs dont le signal est exploité pour déterminer des propriétés de fruits et en particulier leur teneur en sucre.

Le document US2006/0118726 décrit également un dispositif de mesure optique de la qualité de fruits ou de légumes. Il met en oeuvre une technique d'analyse spectrale distinguant une mesure "réelle", une mesure "noire" et une mesure de "référence".

Or, on constate que de nombreux utilisateurs ressentent aujourd'hui le besoin de pouvoir réaliser de telles analyses rapidement, directement sur le terrain. Dans le domaine agroalimentaire, on peut ainsi citer la viticulture, où le suivi de la maturité du raisin en mesurant l'évolution de la teneur en sucre, en acide ou encore en anthocyane, de préférence sur pied, permet de déterminer une date optimale de récolte. On peut encore citer la viniculture où il est intéressant de mesurer l'évolution de la teneur en alcool afin de suivre le processus de fermentation. L'oléiculture est un autre domaine d'application, où un suivi de la maturité de l'olive par la mesure de la teneur en huile et en eau d'échantillon d'olive, si possible sur l'arbre, permet de déterminer un moment opportun de récolte. De manière similaire, la mesure de la teneur en sucre d'un fruit, tel que : pomme, poire, melon, ..., permet d'évaluer la maturité des fruits cultivés et de déterminer une date optimale de récolte. On peut citer encore les grandes cultures céréalières où une mesure de la teneur en azote dans une feuille permet la détermination d'un apport d'engrais azoté raisonné.

Les exemples ici cités proviennent de l'industrie agroalimentaire, mais l'on pourrait en citer d'autres issus d'autres industries, telles que pétrochimie (analyse de teneurs en hydrocarbures), diagnostic médical (mesure du taux de sucre pour un diabétique), etc.

La viticulture, est soumise aujourd'hui à une exigence croissante d'optimisation des rendements et de la qualité, afin de mieux adapter les productions aux demandes du marché et aux attentes des consommateurs. Pour répondre à cette exigence, une solution consiste à mieux évaluer les critères de maturité du raisin afin de prédire une date optimale de récolte.

Depuis la véraison jusqu'à la maturité des raisins, des tests de suivi de maturité sont réalisés. Ces tests nécessitent actuellement des prélèvements d'échantillons de grappe ou de baie sur différentes parcelles. Ces échantillons sont ensuite transmis à des laboratoires d'analyse. Les mesures ainsi réalisées sont contraignantes et onéreuses. Elles nécessitent la destruction des échantillons. La collecte d'échantillon est longue. Les analyses en laboratoire sont coûteuses. De plus, ce mode opératoire ne permet de connaître qu'à posteriori l'état d'avancement de la maturité.

Il apparaît donc un besoin pour un appareil portable, autonome, utilisable sur le terrain, capable de collecter les mesures de teneurs de certains composés choisis, directement sur un produit.

La présente invention permet de répondre à ce besoin en proposant un spectromètre optique autonome et portatif, capable de mesurer, directement sur le produit, sans nécessiter sa destruction, les teneurs d'un ou plusieurs composés configurables.

L'invention a pour objet un spectromètre optique tel que défini par la revendication 1.

D'autres caractéristiques possibles du spectromètre apparaissent dans les revendications 2 à 10.

L'invention concerne encore un procédé d'utilisation d'un spectromètre optique tel que défini par la revendication 11.

D'autres caractéristiques possibles du procédé apparaissent dans les revendications subséquentes dépendant de la revendication 11.

Un avantage du spectromètre et du procédé selon l'invention est de proposer un appareil portatif et autonome apte à être utilisé sur le terrain afin de produire, de manière non destructive, des mesures immédiatement exploitables, avec une précision comparable à celle des appareils de laboratoire et permettant de mesurer des teneurs en composés, y compris très faibles.

D'autres caractéristiques, détails et avantages de l'invention ressortiront plus clairement de la description détaillée donnée ci-après à titre indicatif en relation avec des dessins sur lesquels :

- la figure 1 présente un schéma détaillant les principes optiques utilisés,
- la figure 2 présente un schéma directeur de la géométrie d'un spectromètre selon l'invention,
- la figure 3 présente une vue en coupe d'un spectromètre selon un premier mode de réalisation,
- la figure 4 détaille l'ouverture du spectromètre de la figure 3,
- la figure 5 présente une vue en coupe d'un spectromètre selon un second mode de réalisation,
- la figure 6 illustre un spectre d'éclairage,
- la figure 7 illustre un spectre rétro diffusé,
- les figures 8 à 11 illustrent quatre conformations de chambre de mesure,
- les figures 12 et 13 illustrent un dispositif de montage des capteurs optiques selon un premier mode de réalisation en vue perspective respectivement monté et éclaté,
- les figures 14 et 15 illustrent un dispositif de montage des capteurs optiques selon un second mode de réalisation en vue perspective respectivement monté et éclaté,
- la figure 16 présente un schéma d'une chaîne de mesure,
- la figure 17 présente un schéma du système informatique du spectromètre,
- la figure 18 présente une vue perspective du spectromètre,
- la figure 19 présente une vue perspective éclatée du même spectromètre,

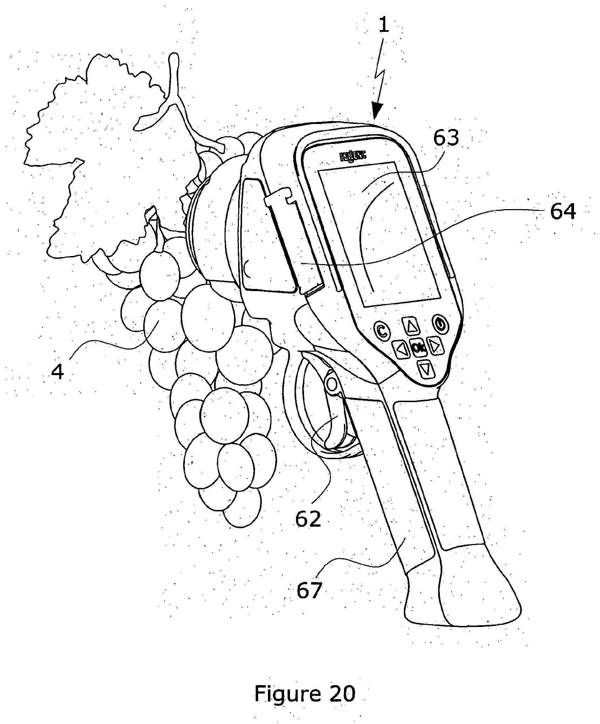

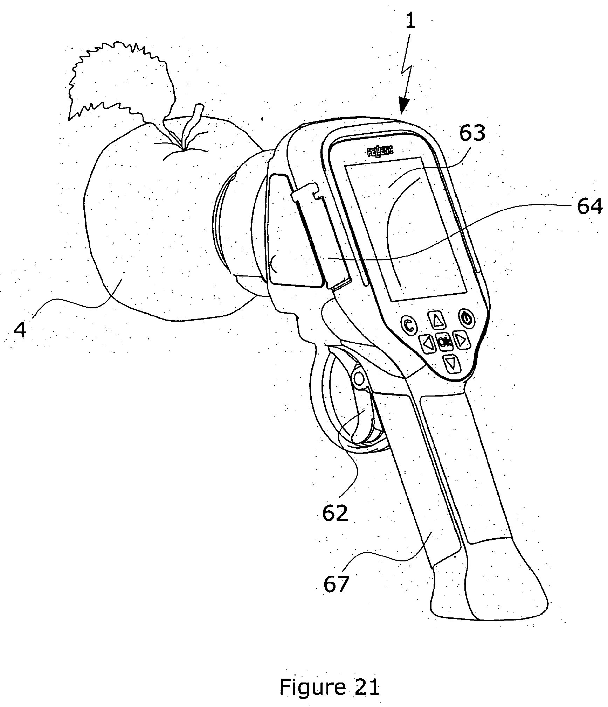

- les figures 20 et 21 présentent deux exemples d'utilisation du spectromètre,
- la figure 22 figure un organigramme d'une séquence de mesure,
- les figures 23 et 24 indiquent les coefficients caractéristiques de deux applications.

On se réfère auxdits dessins pour décrire des exemples avantageux mais non limitatifs de réalisation du spectromètre optique et de mise en oeuvre du procédé selon l'invention.

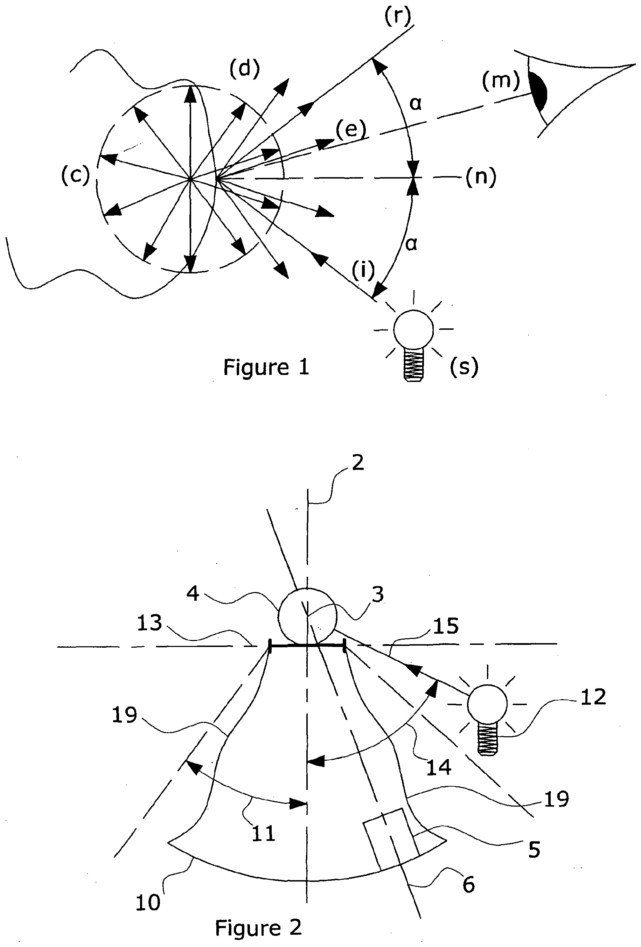

La figure 1 illustre un principe optique. Un corps c éclairé par un rayon de lumière incident i, issu d'une source lumineuse s, réfléchit cette lumière selon au moins trois modes.

Dans un premier mode "spéculaire" un rayon réfléchi r, est principalement concentré selon une direction privilégiée. Cette direction est, telle que déterminée par les lois de Descartes, symétrique relativement à la normale n à la surface du corps c au point d'impact du rayon incident i. L'angle entre le rayon incident i et la normale n est égal à l'angle entre la normale n et le rayon réfléchi spéculaire r.

Dans un second mode "diffus" la lumière est rétro diffusée d de manière sensiblement homogène dans toutes les directions de l'espace. Ce second mode est mis à profit par l'invention. -Il présente l'avantage de permettre une mesure en plaçant un capteur m dans toute direction de l'espace en étant assuré de pouvoir observer un même signal. Un inconvénient est que l'intensité de ce signal est faible relativement au signal spéculaire réfléchi r. Aussi l'invention présente plusieurs caractéristiques permettant d'améliorer la qualité de la mesure afin de compenser la relative faiblesse du signal reçu. Il convient cependant, pour observer ce mode diffus, de ne pas placer un capteur dans la direction r de réflexion spéculaire afin de ne pas risquer de l'aveugler/saturer, du fait des différences d'ordre de grandeurs des intensités lumineuses.

Au contraire du mode spéculaire où la réflexion s'effectue en surface de l'objet, le mode diffus s'accompagne d'une certaine pénétration de la lumière à l'intérieur du corps c. En fonction de la composition du corps c, le spectre de lumière se trouve modifié du fait d'atténuations différentielles, selon les longueurs d'onde. Ainsi, une propriété essentielle est qu'un signal rétro diffusé par un échantillon voit son spectre de longueurs d'onde modifié en fonction des composés présents dans l'échantillon qui absorbent de manière sélective certaines longueurs d'ondes caractéristiques desdits composés.

Le principe de mesure du spectromètre 1 selon l'invention met à profit cette propriété. Il consiste à éclairer de manière active, avec une source lumineuse de spectre connu 21, un échantillon 4 disposé dans une zone cible 3, à mesurer la lumière réfléchie selon le mode rétro diffusé par l'échantillon 4 au moyen de capteurs optiques 5 afin d'identifier et de caractériser le spectre rétro diffusé 22. Ensuite, à partir de cette mesure, une modification du spectre rétro diffusé 22 relativement au spectre d'éclairage 21 est déterminée. L'analyse de cette modification, en quantifiant les atténuations observables selon certaines longueurs d'onde caractéristiques permet de déduire les teneurs de certains composés constitutifs de l'échantillon 4.

Un troisième mode de réflexion « de surface », dû aux micro rugosités de surface, existe encore et est utilisé pour l'observation de la couleur.

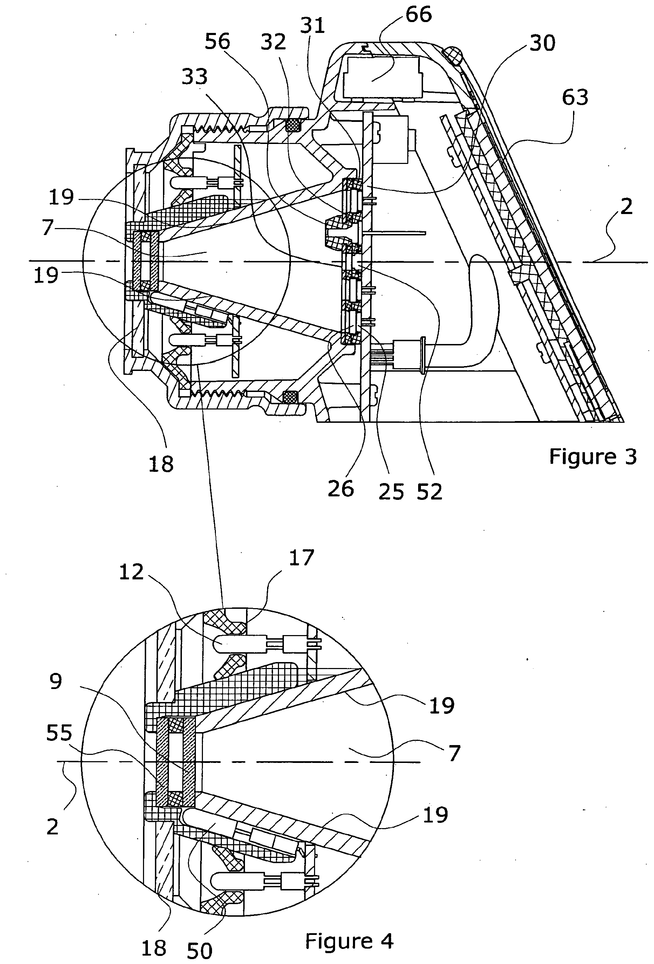

La figure 2 présente un synoptique de définition de la géométrie d'un spectromètre optique 1 selon l'invention, tandis qu'est représenté aux figures 3 et 4, respectivement 5 et 6, un premier mode, respectivement un second mode, de réalisation illustratif d'un tel spectromètre optique 1. Le spectromètre optique 1 est organisé autour d'un axe optique 2. Une zone cible 3, centrée sur ledit axe optique 2, en périphérie du spectromètre est libre et accessible afin de permettre l'introduction d'un échantillon 4 à analyser. La taille de cette zone cible 3 est adaptée en fonction des produits que l'on souhaite analyser. Son centre 3 est situé à l'intersection de l'axe optique 2 et des axes 6 des capteurs optiques 5. Son étendue spatiale varie en fonction des types d'échantillon 4 traités et des caractéristiques optiques du spectromètre. L'échantillon 4 peut être un solide ou un liquide.

Le spectromètre optique 1 comprend une pluralité de capteurs optiques 5. Chaque capteur optique 5 vise la zone cible 3. Autrement dit, son axe optique 6 passe par la zone cible 3.

Le spectromètre optique 1 comprend encore une chambre de mesure 7 globalement opaque à la lumière, à l'exception d'une ouverture 8.

Cette ouverture 8 est centrée sur l'axe optique 2 et constitue l'unique voie d'entrée de lumière de l'extérieur vers l'intérieur de la chambre de mesure 7. La zone cible 3 est proche de l'ouverture 8, tout en restant extérieure à ladite ouverture 8 et à la chambre de mesure 7.

L'ouverture 8 est encore totalement obstruée par au moins un filtre diffuseur 9. Ainsi toute lumière entrant dans la chambre de mesure 7 depuis l'extérieur se voit diffusée par la traversée dudit filtre diffuseur 9. Elle est ainsi homogénéisée dans l'intérieur de la chambre de mesure 7. Une mesure de lumière peut ainsi être réalisée de manière identique en tout point de la chambre de mesure 7, ce qui autorise une grande latitude de positionnement pour un capteur optique 5, à l'intérieur de la chambre de mesure 7.

Afin d'optimiser la puissance lumineuse entrant dans la chambre de mesure 7, le filtre diffuseur 9 présente une très faible atténuation. La zone cible 3 est limitée par la surface externe du filtre diffuseur 9. La surface externe du filtre diffuseur 9 fournit aussi une surface d'appui permettant une mise en contact de l'échantillon 4 pendant une séquence de mesure. Le filtre diffuseur 9 assure encore une protection de l'intérieur de la chambre de mesure 7 contre les introductions éventuelles de corps étrangers solides ou liquides.

La chambre de mesure 7 comprend encore un fond intérieur 10. Ce fond intérieur 10 est centré sur l'axe optique 2 et présente une surface apte à accueillir la pluralité de capteurs optiques 5.

Afin d'éclairer l'échantillon 4 de manière active, avec une source lumineuse de spectre 21 connu, le spectromètre 1 comprend un dispositif d'éclairage principal 12. Ce dispositif d'éclairage principal 12 est apte à éclairer l'échantillon 4. Il est placé à l'extérieur de la chambre de mesure 7.

La fonction de l'éclairage principal 12 est d'éclairer l'échantillon 4, de manière suffisamment puissante afin de produire une réflexion diffuse qui puisse être mesurée par la pluralité de capteurs optiques 5.

La direction de l'éclairage principal 12 relativement à l'axe optique 2, afin de produire une réflexion diffuse en direction de la chambre de mesure 7, peut être quelconque puisque la réflexion diffuse est omnidirectionnelle. Il est cependant préférable que l'éclairage principal 12 n'éclaire pas en vue directe l'ouverture 8, afin de ne pas perturber les capteurs optiques 5 par un signal beaucoup plus intense que celui, rétro diffusé, que l'on souhaite mesurer. Pour cela le dispositif d'éclairage principal 12 doit au moins être disposé, du côté opposé à la zone cible 3, relativement au plan 13 perpendiculaire à l'axe optique 2 et passant par le filtre diffuseur 9 (ledit plan coïncide avec le plan de l'ouverture 8 et avec le plan de pose de l'échantillon 4).

Puisque l'on veut exploiter la réflexion diffuse, d'intensité plus faible relativement à la réflexion spéculaire, il faut garantir qu'une réflexion spéculaire ne pénètre pas dans la chambre de mesure 7 (via l'ouverture 8), ce qui perturberait sensiblement la mesure par aveuglement des capteurs optiques 5. Ceci est obtenu par le positionnement précédemment décrit de l'éclairage principal 12, en coopération avec un positionnement de l'échantillon plaqué contre le plan 13. La présence d'un filtre diffuseur 9, en plus de sa fonction principale précédemment décrite, assure une fonction secondaire de diffusion d'une éventuelle réflexion spéculaire pénétrant dans la chambre de mesure 7. Cette diffusion atténue grandement les conséquences d'une telle réflexion. En dernier recours, un test d'acceptation, décrit plus loin, permet, le cas échéant, de détecter au niveau de la mesure, la présence d'une anomalie causée par une telle réflexion spéculaire.

Entre l'émission par le dispositif d'éclairage 12 et la rétro diffusion, les longueurs d'onde ne sont pas modifiées, seules l'intensité de la lumière est plus ou moins atténuée selon les longueurs d'onde particulières d'absorption des composés constituants l'échantillon 4. Le spectre 22 de la lumière rétro diffusée dans la chambre de mesure 7 est analysé au moyen des capteurs optiques 5. Ces derniers réalisent avantageusement des mesures d'intensité selon certaines longueurs d'onde discrètes particulièrement choisies. Il est ainsi nécessaire que le spectre 21 de la lumière émise par le dispositif d'éclairage principal 12 contienne toutes les longueurs d'ondes pour lesquelles on souhaite mesurer une atténuation au moyen des capteurs optiques 5. Un premier moyen simple consiste à employer un dispositif d'éclairage principal 12 présentant un large spectre incluant toutes les longueurs d'ondes particulièrement choisies. On remarque cependant qu'un éclairage ne comportant a minima que lesdites longueurs d'onde particulièrement choisies est suffisant. Il est cependant plus aisé de réaliser un éclairage formé d'un large spectre couvrant typiquement les longueurs d'ondes comprises entre 300 et 1800 nm. Un tel spectre couvre ainsi toutes les longueurs d'onde correspondant à tous les composés dont on peut souhaiter mesurer la teneur.

Dans le cas d'une application illustrative appliquée à la maturation du raisin, un spectre compris entre 300 et 1100 nm est suffisant pour couvrir les longueurs d'ondes choisies.

Un spectre large de ce type peut être obtenu par tout moyen d'éclairage classique, tel que : ampoule à incandescence, ampoule à gaz, diode électroluminescente (DEL) ou toute combinaison de ces composants.

Le dispositif d'éclairage principal 12 est avantageusement disposé de manière annulaire autour de l'axe optique 2 afin d'augmenter la quantité de lumière éclairant l'échantillon 4. Il est avantageux d'augmenter au maximum cette quantité de lumière, afin d'améliorer la sensibilité du spectromètre 1, malgré un faible « rendement » de la rétro diffusion. Un réflecteur 17, par exemple métallisé et annulaire, peut encore être utilisé afin de concentrer le flux lumineux de l'éclairage en direction de la zone cible 3 et de l'échantillon 4.

Dans un mode de réalisation avantageux, le dispositif d'éclairage principal 12 est protégé par une paroi de protection 18. Afin de ne pas perdre de puissance lumineuse, cette paroi est avantageusement translucide et optiquement neutre. Elle permet cependant de protéger les composants de l'éclairage principal 12 contre les corps étrangers. Cette paroi de protection 18 est, par exemple, réalisée en verre.

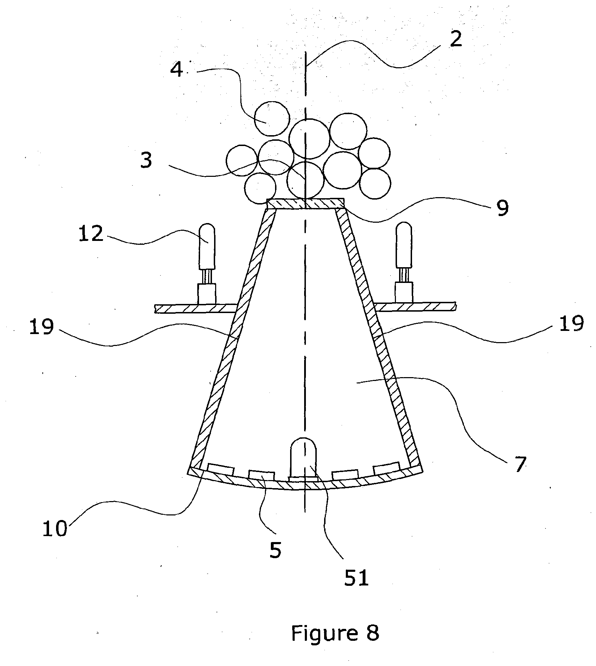

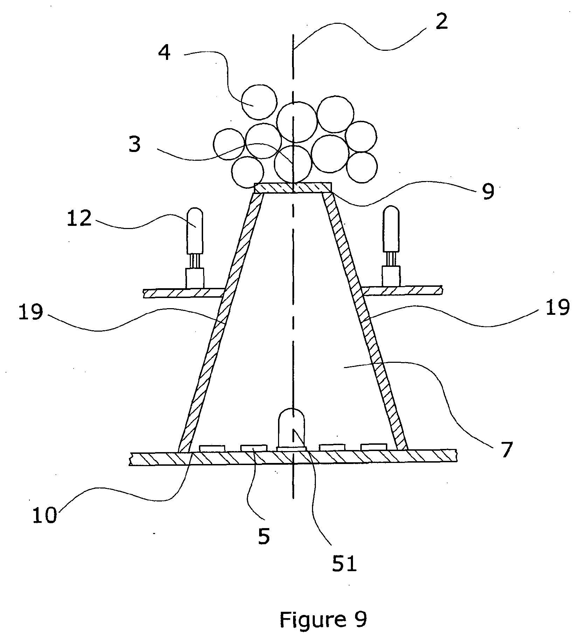

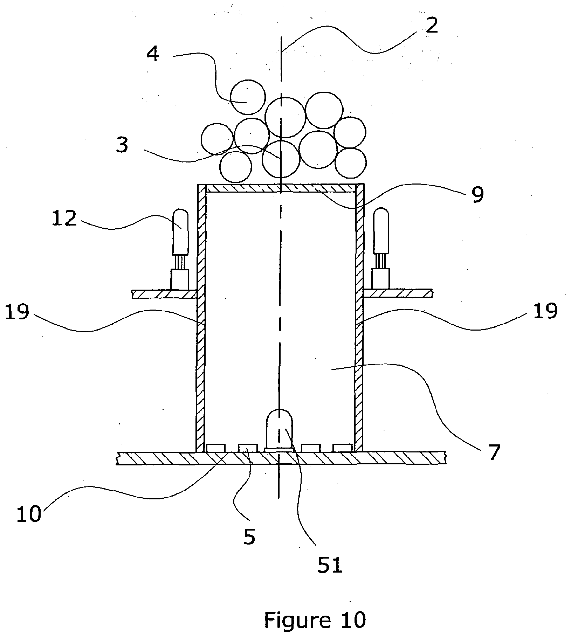

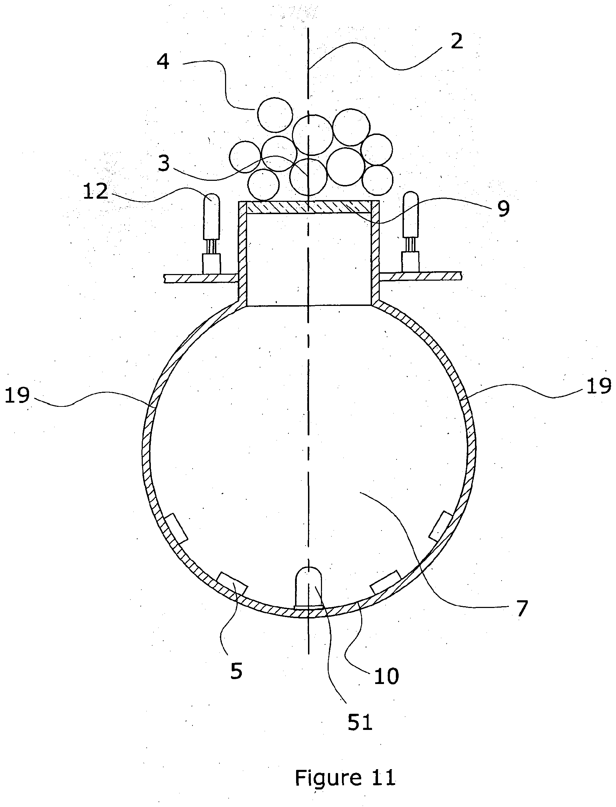

La fonction de la chambre de mesure 7 est d'accueillir les capteurs optiques 5. Cependant ces capteurs optiques 5 ne peuvent être tous disposés en un même endroit idéal de mesure. Ceci est compensé par une homogénéisation de tout signal lumineux entrant dans la chambre de mesure 7, réalisée par au moins un filtre diffuseur 9. Pour que ledit filtre diffuseur 9 soit efficace dans sa fonction de diffusion, la forme de la paroi latérale interne 19 de la chambre de mesure 7 peut être quelconque à condition d'être au moins incluse dans une enveloppe de forme conique dont l'angle d'ouverture 20 reste inférieur ou égal à l'angle de diffusion 11 du filtre diffuseur 9. Ainsi, comme illustré aux figures 8 à 11, cette paroi latérale 19 peut être conique, cylindrique, sphérique ou autre.

L'effet d'homogénéisation par diffusion est encore amélioré par un mode de réalisation où ladite paroi interne 19 de la chambre de mesure 7 est optiquement absorbante. Ceci peut être réalisé, de manière connue, par un matériau ou un revêtement adapté.

Il a été mentionné que les capteurs optiques 5 visent la zone cible 3. Afin de permettre une mise en place aisée des capteurs optiques 5 réalisant cette condition, un premier mode de réalisation utilise idéalement un fond intérieur 10 de la chambre de mesure 7 présentant une forme en calotte sphérique, le centre de cette sphère étant situé au niveau de la zone cible 3. Ainsi un capteur optique 5, monté à plat sur le fond intérieur 10, a de fait son axe optique 6, normal à la surface de la calotte sphérique, aligné avec la zone cible 3.

Cependant la réalisation d'un fond intérieur 10 sphérique est complexe et on lui préfère un fond plan, plus simple. Dans ce cas cependant, l'axe optique 6 d'un capteur optique 5 se trouve d'autant plus désaligné d'avec la zone cible 3 que le capteur optique 5 est monté loin de l'axe optique 2. Deux techniques seront décrites ci-après afin de corriger / compenser ce désalignement axial.

Afin d'optimiser la sensibilité du spectromètre 1, il convient d'augmenter au maximum l'intensité de lumière reçue à l'intérieur de la chambre de mesure 7. En plus de retenir un filtre diffuseur 9 à faible atténuation, et un éclairage principal 12 puissant et muni d'un réflecteur, la géométrie de la chambre de mesure 7 peut aussi être optimisée. La proportion de ladite chambre de mesure 7 est avantageusement conçue de manière à présenter un gain photométrique G maximal. Le gain photométrique G est défini par la formule : $G = 100 . \left( \dfrac{\Phi}{L} \right)^2$ , où $\Phi$ est le diamètre de l'ouverture 8, et *L* est la distance entre ladite ouverture 8 et le fond intérieur 10. Pour augmenter G il est possible soit d'augmenter $\Phi$, soit de réduire *L*. Dans la pratique on retiendra avantageusement une valeur de G au moins égale à 4.

Le diamètre $\Phi$ est lié aux dimensions typiques des échantillons 4. La taille du fond intérieur 10 dépend du nombre de capteurs optiques 5 utilisés. Ce nombre dépend des teneurs en composés retenus que l'on souhaite mesurer.

L'angle d'ouverture de la chambre de mesure est au moins égal à l'angle de diffusion du filtre diffuseur 9. Une ouverture 11 de la chambre de mesure 7 trop importante est préjudiciable en ce qu'une partie de la lumière entrant dans ladite chambre de mesure 7 est perdue. Avantageusement, l'ouverture de la chambre est choisie supérieure mais presque égale à l'angle de diffusion du filtre diffuseur 9. La géométrie de la chambre de mesure 7 est ainsi déterminée, et l'on choisira avantageusement une longueur L minimale respectant la précédente condition d'angle.

Ainsi, dans le cas d'application au raisin, dont on souhaite mesurer des grappes, la dispersion importante des tailles d'échantillon a conduit expérimentalement à retenir un diamètre optimum $\Phi$ de 10 mm. Les composés retenus, dans ce cas, conduisent à employer 13 capteurs optiques 5, de dimension 5x5 mm que l'on place sur un fond intérieur 10 de diamètre 36,5 mm. Un filtre diffuseur 9 présentant un angle de diffusion de 40° conduit à une distance L minimale de 50 mm, soit un gain photométrique sensiblement égal à 4.

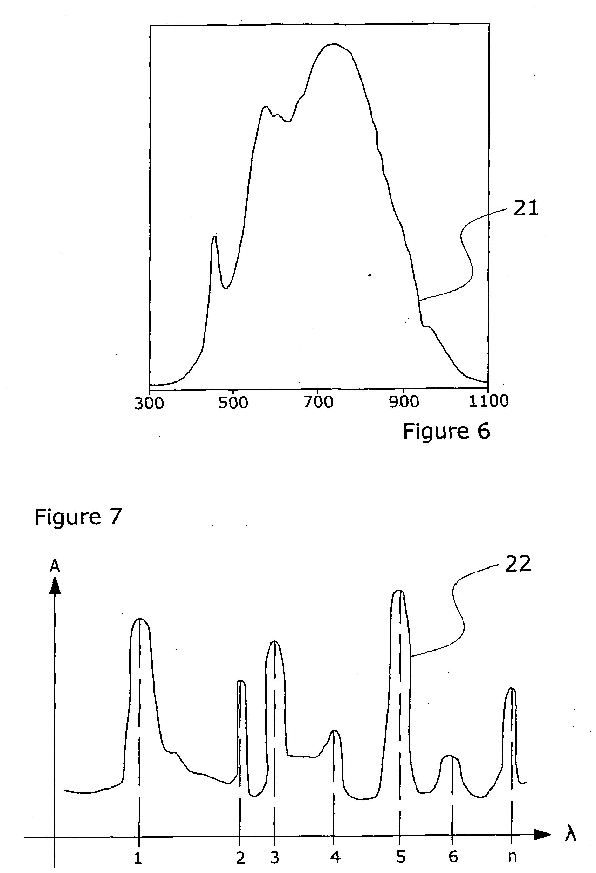

Afin d'analyse du spectre de lumière rétro réfléchie, la chambre de mesure 7, et particulièrement son fond intérieur 10, est équipée de capteurs optiques 5. Selon une caractéristique importante et avantageuse de l'invention, ledit spectre rétro réfléchi 22, dont un exemple est illustré à la figure 7 comparativement au spectre d'éclairage 21 illustré à la figure 6, est analysé au moyen de mesures d'intensité de lumière. Selon un mode de réalisation particulier, ces mesures sont réalisées sur un ensemble de valeurs discrètes de longueurs d'ondes choisies en fonction de l'ensemble des composés dont l'on souhaite déterminer la teneur. A chacune de ces longueurs d'onde est associé un capteur optique 5 mesurant l'intensité lumineuse dans une bande étroite centrée sur ladite longueur d'onde. Le choix desdites longueurs d'onde est avantageusement obtenu par une optimisation prenant globalement en compte l'ensemble des composés dont l'on souhaite déterminer la teneur et qui est décrite ci-après.

Afin de réaliser un tel capteur optique 5 dédié à une longueur d'onde ainsi choisie, ce capteur optique 5 comprend par exemple une photodiode 25 et un filtre optique 26 disposé devant une surface sensible de ladite photodiode 25. Ainsi ladite photodiode 25 mesure uniquement l'intensité lumineuse reçue dans une bande autour de ladite longueur d'onde. La photodiode peut être une photodiode de type Silicium (Si) ou encore Indium Gallium Arsenide (InGaAs). Elle est apte à délivrer en sortie un signal électrique analogique indicatif de l'intensité lumineuse reçue.

Selon un mode de réalisation avantageux, la photodiode 25 est une photodiode à grande surface réceptrice, afin d'améliorer la quantité de lumière reçue. Ceci contribue à améliorer le rapport signal sur bruit et la sensibilité du spectromètre 1.

En associant les différentes caractéristiques géométriques, optiques et électroniques du spectromètre, il est possible d'obtenir un rapport signal sur bruit des mesures d'intensité lumineuse supérieur ou égal à 6000, qui rend possible la détermination de teneur en composé très faible.

Le filtre optique 26 est avantageusement un filtre passe bande étroite centré sur ladite longueur d'onde. Des filtres présentant une largeur de bande de 4 nm permettent d'obtenir un résultat satisfaisant. Avantageusement la dimension du filtre optique 26 est suffisamment grande pour permette de couvrir le plus complètement possible la surface sensible de la photodiode 25 à laquelle il est associé afin de maximiser le rendement lumineux du capteur optique 5. Le filtre optique 26 est avantageusement plaqué au plus près contre la surface sensible de la photodiode 25.

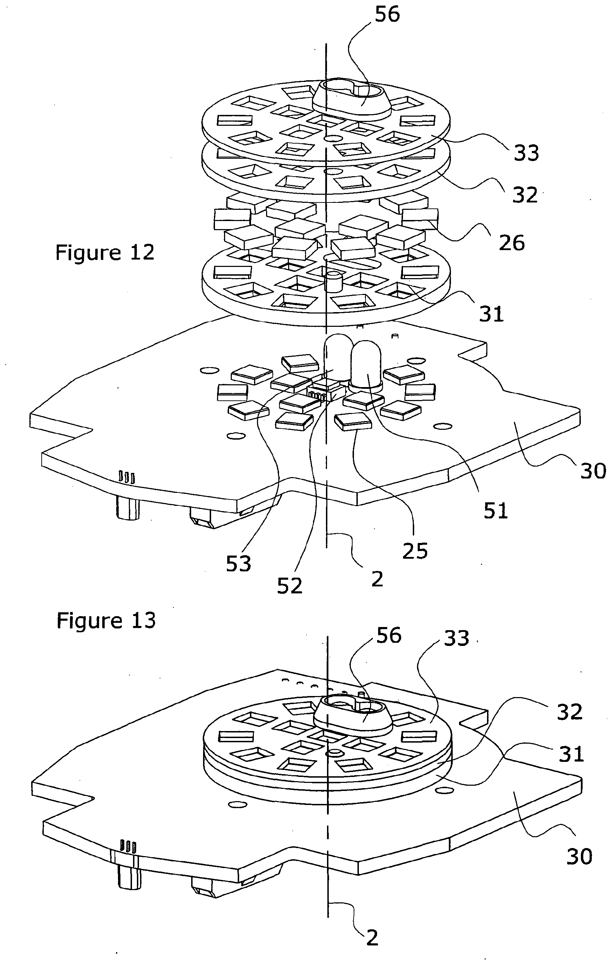

Selon un mode de réalisation avantageux illustré aux figures 12 à 15, les capteurs optiques 5 sont assemblés et positionnés par un assemblage multi pièces.

Selon un premier exemple de réalisation, illustré aux figures 12 et 13, une première pièce support 30 rigide sensiblement plane accueille les photodiodes 25 et assure leur positionnement précis. Avantageusement, cette pièce 30 peut être un circuit imprimé 30. Les photodiodes sont alors positionnées par soudage et le circuit imprimé assure encore le câblage des photodiodes. Une seconde pièce entretoise 31 rigide sensiblement plane, dans laquelle sont découpés des logements aptes à accueillir les filtres optiques 26, assure leur positionnement relativement aux photodiodes 25 dans le plan de l'assemblage. Une troisième pièce intercalaire 32 sensiblement plane, réalisée dans un matériau élastique, est découpée de manière à se superposer à la pièce support 31. L'épaisseur cumulée de la pièce support 31 et de la pièce intercalaire 32 est telle qu'elle soit supérieure à l'épaisseur des filtres optiques 26 lorsque la pièce intercalaire 32 est au repos, et inférieure à ladite épaisseur lorsque la pièce intercalaire 32 est comprimée. Une quatrième pièce de serrage 33 assure le maintien de l'ensemble. La quatrième pièce de serrage 33 comporte, au niveau des filtres optiques 26, des découpes découvrant au moins la surface sensible de chaque photodiode 25, mais couvrant la périphérie de chaque filtre optique 26 afin d'assurer son maintien. La compression de la pièce intercalaire 32 assure un rattrapage de jeu, et un plaquage des filtres optiques 26 contre les photodiodes 25, selon la direction de l'axe 6 du capteur optique, perpendiculaire au plan l'assemblage.

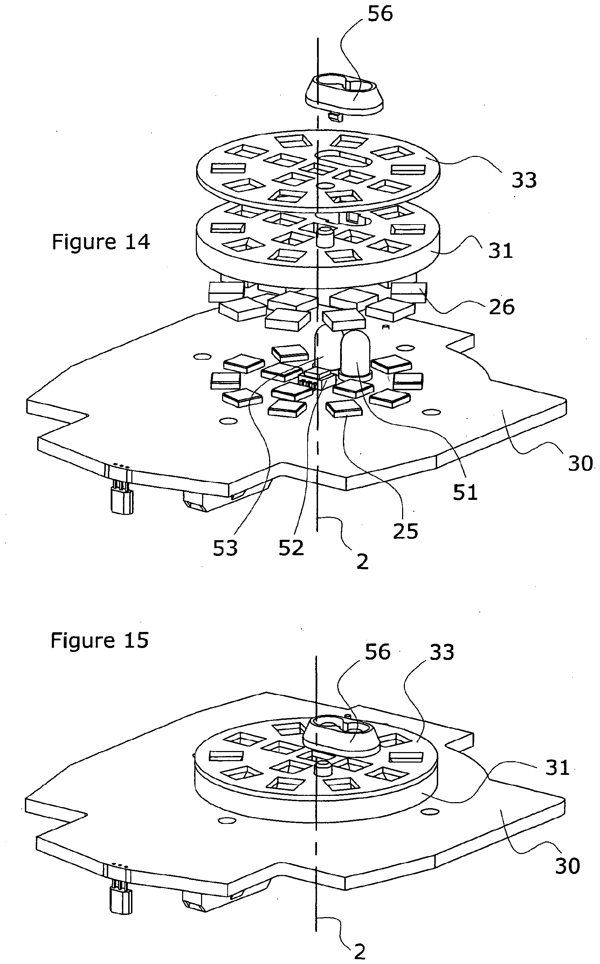

Selon un mode de réalisation alternatif, illustré aux figures 14 et 15, les pièces entretoise 31 et intercalaire 32 sont confondues en une unique pièce 31 élastique assurant leurs fonctions.

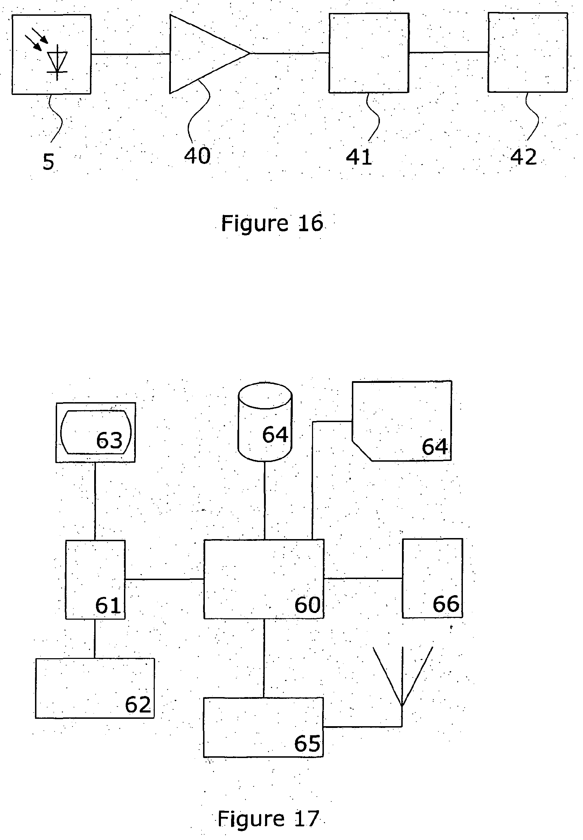

La figure 16 illustre un mode de réalisation de la chaîne de traitement associée à un capteur optique 5. Chaque capteur optique 5 est associé à un amplificateur/filtre 40, 41, afin de traiter le signal électrique de sortie du capteur optique 5. Ce filtre de traitement de signal comprend au moins un amplificateur 40. Avantageusement le gain de chaque amplificateur 40, associé à un capteur optique 5, est réglable séparément, afin de pouvoir maximiser l'excursion de la mesure de l'intensité lumineuse observable sur ce capteur optique 5, en conditions normales de mesure. Pour chaque capteur optique 5, en fonction des composés dont on souhaite déterminer la teneur, et de la population typique d'échantillon probable, il est possible de déterminer, le cas échéant avec une marge de sécurité, une valeur maximale d'intensité lumineuse observable. Le gain de l'amplificateur 40 est alors réglé pour que cette valeur maximale d'intensité corresponde à la valeur maximale de la mesure acceptable par un dispositif d'acquisition 42 en aval. Ceci permet d'exploiter au maximum le signal mesuré en profitant de la dynamique du dispositif de traitement 42 en aval afin d'obtenir une grande sensibilité et un meilleur rapport signal sur bruit.

La chaîne de traitement de capteur optique peut encore comprendre d'autres composants. Il est ainsi possible d'utiliser des filtres 41 ou d'autres composants, afin de réaliser tout prétraitement du signal électrique de mesure. Le dispositif de traitement 42 est avantageusement un convertisseur analogique numérique 42 afin d'interfaçage avec une unité logique de traitement 60 décrite plus loin. En fonction du nombre de capteurs optiques 5, il est encore possible d'employer un multiplexeur afin de multiplexer lesdits capteurs optiques 5 avec ladite unité logique de traitement 60.

Comme exposé précédemment, il convient que les capteurs optiques 5 visent la zone cible 3. Un premier mode de réalisation avec un fond intérieur 10 sphérique remplit cette condition, mais s'avère difficile à mettre en oeuvre, notamment dans le mode de réalisation préférentiel utilisant un circuit imprimé plan. Un fond intérieur 10 plat est donc préféré. Cependant sur un fond support plat, l'axe 6 d'un capteur optique 5 ne vise plus la zone cible 3, et est d'autant plus désaligné par rapport à une direction visant la zone cible 3 que le capteur optique 5 est monté loin de l'axe optique 2.

Un premier mode de réalisation consiste à remédier à ce désalignement par une correction angulaire, par exemple au moyen d'un support biais, radialement disposé, pour chaque capteur optique 5, le biais étant fonction de la distance à l'axe optique 2, afin de reproduire l'angle que le capteur optique 5 présenterait, monté sur une calotte sphérique. Cependant une telle correction est difficile à mettre en oeuvre compte tenu des faibles angles à considérer.

Un second mode de réalisation consiste à corriger non plus ledit désalignement mais sa conséquence. Un désalignement axial modifie de manière connue la longueur d'onde mesurée du signal lumineux en la décalant vers le bleu. L'angle de désalignement axial étant connu, sa conséquence sur la mesure du signal lumineux peut être corrigée en décalant la longueur d'onde associée au capteur optique 5 d'une valeur correspondant audit angle de désalignement. Ce décalage est réalisé par le choix du filtre optique 26 lors de sa conception. Ainsi pour une longueur d'onde λ déterminée en fonction des composés, le capteur optique est "réglé" sur une longueur d'onde décalée λ' fonction de la longueur d'onde λ et de la distance (ou angle de désalignement de l'axe 6) du capteur optique 5 relativement à l'axe optique 2.

Les longueurs d'onde associées audits capteurs optiques 5 permettent de caractériser le spectre de lumière rétro diffusée. Le nombre et les valeurs des longueurs d'onde retenus peuvent être quelconques. Cependant un choix judicieux de ce nombre et de ces valeurs permet d'améliorer nettement la qualité des mesures.

Ainsi, selon une méthode préférentielle le nombre de longueurs d'onde et les valeurs particulières de ces longueurs d'onde peuvent être globalement choisis en fonction du jeu de composés dont on souhaite déterminer les teneurs.

Un exemple de méthode de détermination des longueurs d'ondes est basé sur une optimisation globale obtenue en maximisant une fonction objectif ou de performance. On considère un ensemble étendu de longueurs d'onde candidates $\lambda_1..\lambda_m$. Le cardinal m de cet ensemble peut être très grand. Ainsi il est possible de partir en incluant toutes les longueurs d'onde du spectre retenu, avec un pas donné de 1, 2 ou quelques nm, ou encore de toutes les longueurs d'onde commercialement disponibles. Une sélection S quelconque de n, n<m, longueurs d'onde est considérée dans cet ensemble, et la fonction objectif est calculée pour cette sélection S. La fonction objectif F est choisie telle que sa valeur F(S) soit maximale lorsque les capteurs optiques associés à la sélection S de longueurs d'onde réalisent une erreur minimale et ce avec un nombre minimum de longueurs d'onde. Un exemple de fonction est donné par la formule :

$$F(S) = \sum_{i=1}^{n} \frac{E_i}{S_i} \ ,$$ où i parcourt, de 1 à k, les composés à prédire, $E_i$ est l'erreur de prédiction obtenue pour le i<sup>ème</sup> composé à prédire, et $S_i$ est l'écart type de concentration de ce i<sup>ème</sup> composé à prédire.

Il est ensuite itéré sur les différentes sélections S. Une méthode simpliste itère sur toutes les sélections S possibles. L'homme du métier des méthodes d'optimisation globales connaît des heuristiques permettant de réduire sensiblement les sélections S retenues et ainsi la taille de l'espace de recherche.

Pour l'exemple illustratif appliqué au raisin, le choix des quatre composés : eau, sucre, polyphénols anthocyanes, acide, permet de déterminer une série optimale de 13 longueurs d'onde « théoriques » suivantes : 440, 520, 665, 690, 740, 770, 805, 840, 875, 910, 945, 980 et 1015 nm.

Compte tenu du fait que les dix premiers capteurs optiques (440 à 910 nm) sont disposés sur le circuit imprimé 30 selon une première couronne extérieure et présentent un désalignement résultant de 13,86°, et que les trois derniers capteurs optiques (945 à 1015 nm) sont disposés sur le circuit imprimé 30 selon une seconde couronne intérieure et présentent un désalignement résultant de 6,81°, les longueurs d'onde « corrigées » deviennent : 442, 522, 668, 693, 743, 773, 809, 844, 879, 914, 946, 981 et 1016 nm.

Afin d'améliorer la qualité de mesure du spectromètre 1 objet de l'invention, un dispositif de compensation est avantageusement mis en oeuvre. Pour cela le spectromètre 1 comprend avantageusement un dispositif d'éclairage de référence 50. L'objectif du dispositif d'éclairage de référence 50 est de permettre de réaliser une mesure de référence similaire à la mesure réelle, sauf que la lumière n'est pas rétro diffusée par l'échantillon 4. Ainsi par comparaison des deux mesures obtenues avec et sans atténuation par l'échantillon 4, il est possible de déterminer avec une plus grande précision l'influence de l'échantillon 4, en terme d'atténuation de la lumière.

Ce dispositif d'éclairage de référence 50 est choisi de manière à émettre un spectre de lumière le plus identique possible à celui émis par le dispositif d'éclairage principal 12. Pour cela, le dispositif d'éclairage de référence 50 émet, en conditions d'utilisation comparable, à tout instant, un spectre présentant les même caractéristiques, malgré les éventuels changements de température des dispositifs d'éclairage et malgré leur vieillissement, que le spectre 21 émit par le dispositif principal d'éclairage 12. Un moyen d'atteindre cet objectif est de reproduire à l'identique la structure du dispositif d'éclairage principal 12 en réalisant un dispositif d'éclairage de référence 50 composé du même nombre de constituants (ampoules, DELs, ...), du même type, de même référence et de même lot de fabrication que ceux du dispositif principal d'éclairage 12. Cependant, ceci est surtout vrai pour les lampes à incandescence. Il peut être considéré que les DELs ne sont pas sensibles ni aux variations de température, ni au vieillissement. Dans la pratique un éclairage principal 12 peut comprendre des lampes à incandescence supportées par des DELs afin d'augmenter le flux lumineux, tandis que l'éclairage de référence 50 ne comprend que des lampes à incandescence de même type, même référence et même lot de fabrication.

On peut encore avantageusement, au niveau du procédé d'utilisation, qui sera décrit plus loin, s'assurer de commander des durées comparables d'allumage respectif du dispositif d'éclairage principal 12 et du dispositif d'éclairage de référence 50 afin qu'au moment de leur utilisation pendant les acquisitions, les dispositifs d'éclairage 12, 50 présentent des températures et donc des spectres identiques.

Afin de reproduire, vu des capteurs optiques 5 une lumière comparable à celle issue du dispositif d'éclairage principal 12, le dispositif d'éclairage de référence 50 est disposé hors de la chambre de mesure 7, de manière à ce que sa lumière éclaire le fond intérieur 10 de la chambre de mesure 7, où sont disposés les capteurs optique 5, après avoir été diffusée par le filtre diffuseur 9. Une disposition possible est illustrée aux figures 3 et 4, où le dispositif d'éclairage de référence 50 est disposé sur la tranche du filtre diffuseur 9.

Le spectromètre optique 1 selon l'invention, intègre encore avantageusement, d'autres capteurs optiques afin de réaliser simultanément d'autres mesures sur un même échantillon 4.

Ainsi le spectromètre optique 1 peut comprendre un système de mesure de la couleur 51, 52 de l'échantillon 4. Ce système comprend un troisième dispositif d'éclairage 51 et un capteur optique de couleur 52. Ce système de mesure de la couleur fonctionne, selon le troisième mode de réflexion de surface. Il convient que l'axe d'éclairage de l'échantillon 4 et l'axe de mesure de la couleur soient disposés symétriquement l'un de l'autre par rapport à l'axe optique 2 en supposant que l'échantillon 4, appuyé contre le plan de l'ouverture 8 au niveau de la zone cible 3, présente une normale parallèle audit axe optique 2. Une configuration nécessaire afin de réaliser cette condition, est que, tant le troisième dispositif d'éclairage 51 que le capteur optique de couleur 52, visent la zone cible 3.

Selon un premier mode de réalisation, illustré aux figures 3 et 4, le troisième dispositif d'éclairage 51 et le capteur optique de couleur 52 sont disposés sur le fond intérieur 10.

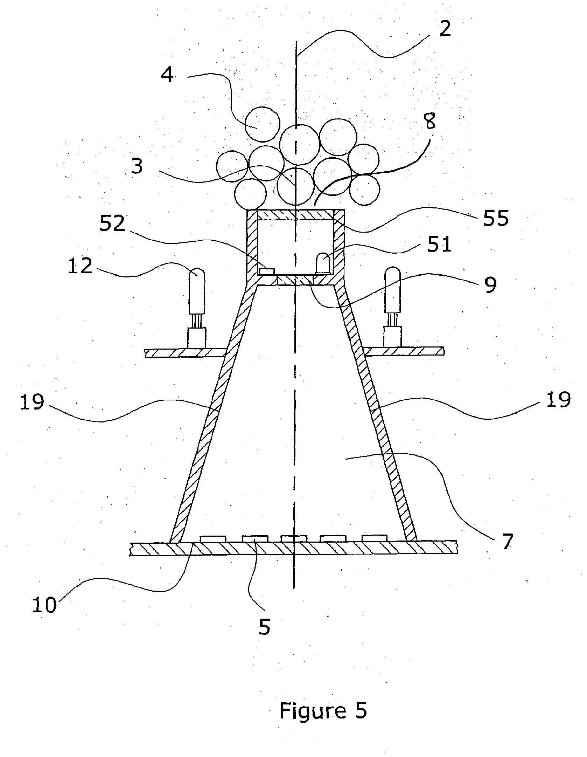

L'ouverture 8 de la chambre de mesure 7 peut être équipée d'une lentille 55 obturant l'ouverture 8 à l'extérieur relativement au filtre diffuseur 9, et le troisième dispositif d'éclairage 51 et le capteur optique de couleur 52 sont disposés entre le filtre diffuseur 9 et la lentille 55. Une telle configuration est par exemple illustrée à la figure 5. La zone cible 3 est, selon ce mode de réalisation, située à l'extérieur de cette lentille 55. Cette lentille 55 remplace alors le filtre diffuseur 9 dans ses fonctions de support de l'échantillon 4 et de protection de la chambre de mesure 7 contre les corps étrangers.

Cette lentille 55 peut être en matériau translucide optiquement neutre tel du verre. Cette lentille 55 peut encore avantageusement être un second filtre diffuseur.

Le troisième dispositif d'éclairage 51 est avantageusement une source de lumière à large spectre, comparable au dispositif d'éclairage principal 12. Ce peut être une DEL émettant un spectre blanc afin de réduire la place occupée. Un cache 56 permet avantageusement de ne pas éclairer directement le capteur optique de couleur 52 proche.

Le capteur optique de couleur 52 mesure, de manière connue, les intensités selon trois couleurs, typiquement rouge, vert et bleu. Le capteur optique de couleur 52 comprend trois capteurs individuels mesurant chacun une des trois couleurs retenues. Ces trois capteurs peuvent encore être intégrés en un composant unique.

Le spectromètre optique 1 peut encore comprendre un système de mesure par fluorescence de l'échantillon 4.

Ce système comprend un quatrième dispositif d'éclairage 53 et un capteur optique spécifique 54. Ce système de mesure par fluorescence fonctionne, par réémission. Le quatrième dispositif d'éclairage 53 et le capteur optique spécifique visent la zone cible 3.

De même, le dispositif de mesure par fluorescence peut, selon un premier mode de réalisation, être disposé sur le fond intérieur 10. Il est avantageux que le quatrième dispositif d'éclairage 53 et le capteur optique spécifique 54 soient tous deux disposés, sur le fond intérieur 10, en coïncidence avec l'axe optique 2.

Selon un second mode de réalisation, le quatrième dispositif d'éclairage 53 et le capteur optique de couleur 54 sont disposés entre le filtre diffuseur 9 et la lentille 55. Une telle configuration est illustrée à la figure 5.

Le principe de la fluorescence consiste à éclairer un échantillon 4 avec une lumière comprenant au moins une première longueur d'onde particulièrement choisie pour provoquer, en présence d'un composé réactif particulier, une émission lumineuse en retour selon une deuxième longueur d'onde. Le quatrième dispositif d'éclairage 53 est alors une source lumineuse monochromatique centrée sur ladite première longueur d'onde ou dont le spectre comprend au moins cette longueur d'onde. Le capteur optique spécifique est alors un capteur sensible à la seconde longueur d'onde, tel par exemple une photodiode équipée d'un filtre optique spectral passe bande centrée sur ladite seconde longueur d'onde.

Ainsi une application particulière, dans l'exemple illustratif de la vigne et du raisin, est la détection d'un champignon parasite nommé botrytis. Ce champignon présente un effet de fluorescence. Lorsqu'il est éclairé par une lumière monochromatique de longueur d'onde centrale 380 nm, le botrytis réagit en réémettant un rayonnement lumineux de longueur d'onde centrale 522 nm, détectable avec un capteur optique spécifique centré sur cette longueur d'onde. Un éclairage comprenant la longueur d'onde 380 nm peut être réalisé au moyen d'une DEL UV produisant un spectre de 380 nm +/-15 nm.

Afin d'orchestrer les différents composants précédents et réaliser les mesures, le spectromètre optique 1, comprend encore une unité de traitement logique 60. Cette dernière est typiquement organisée autour d'un système calculateur à microprocesseur ou microcontrôleur, équipé de périphériques, fonctionnant sous le contrôle d'un logiciel.

Cette unité logique de traitement 60 est interfacée avec les différents dispositifs électriques : dispositifs d'éclairages et capteurs optiques. Ainsi, ladite unité logique de traitement 60 est apte à commander l'allumage sélectif des dispositifs d'éclairages 12, 50, 51, 53. Elle est encore apte à acquérir des mesures issues des différents capteurs optiques et de leurs chaînes 40, 41, 42, de traitement de signal. Elle est encore apte à produire des déterminations par calcul à partir des mesures issues desdits capteurs optiques. Elle est encore apte a stocker et à restituer tant lesdites mesures que lesdites déterminations.

Le spectromètre optique 1 comprend encore une interface homme machine 61, interfacée avec ladite unité de traitement logique 60. Cette interface homme machine 61 comprend au minimum un moyen d'entrée 62, et un moyen de sortie 63. Un moyen d'entrée 62 est typiquement un bouton, un clavier ou une gâchette 62, apte à permettre à un utilisateur du spectromètre optique 1, de déclencher un programme réalisant une séquence de mesures, par exemple selon le procédé qui est décrit ci-après. Un moyen de sortie 63 est typiquement un affichage par exemple un écran 63 apte à afficher les résultats des mesures. D'autre moyens d'entrée peuvent être présents afin, par exemple, de permettre de configurer le spectromètre optique 1.

Parmi les périphériques de l'unité logique de traitement on trouve au moins un moyen de stockage 64. Ce moyen peut comprendre une mémoire interne, le cas échéant amovible, par exemple sous forme d'une carte mémoire flash, afin de pouvoir transmettre des mesures ou déterminations à une autre unité de stockage ou de traitement. Il est possible de redonder ce moyen de stockage afin de garantir une sauvegarde des mesures et déterminations. Ainsi toute mesure peut en parallèle être stockée dans une mémoire interne résidente de type flash ou EEPROM, par exemple, et de manière redondante, par exemple, sur une mémoire amovible de type carte mémoire SD.

On peut aussi trouver un moyen de transmission 65. Ce moyen de transmission 65 peut comprendre une liaison filaire (RS, Ethernet, parallèle, USB,...) ou hertzienne (Wifi, GSM, GPRS, BlueTooth,...). L'avantage d'une liaison hertzienne est de pouvoir transmettre quasi immédiatement des mesures réalisées sur le terrain à une unité de stockage et de traitement centrale.

Ainsi, dans l'exemple illustratif appliqué au raisin, un ou plusieurs spectromètres réalisent des mesures de teneur de composés dans les rangs d'une exploitation viticole. Ces mesures sont transmises à une unité logique de traitement centrale située par exemple au chai, qui compile toutes les mesures pour produire des résultats plus globaux. Ainsi, par exemple, les mesures de teneurs en composés retenus peuvent alimenter une application de prévision de la date optimale de vendange.

Le spectromètre optique 1 peut encore comprendre un dispositif de géo localisation 66. Ce dispositif 66 peut être un récepteur GPS; GPRS ou équivalent. Il est avantageusement interfacé avec l'unité logique de traitement 60, à laquelle il fournit des localisations et, le cas échéant, une date/heure précise. Ainsi, l'unité logique de traitement 60 peut avantageusement associer à une mesure sa localisation géographique et/ou sa date/heure, à l'instant de la mesure ou détermination. Ceci est intéressant afin de dresser des cartographies des mesures en différents endroits d'un établissement ou d'une exploitation.

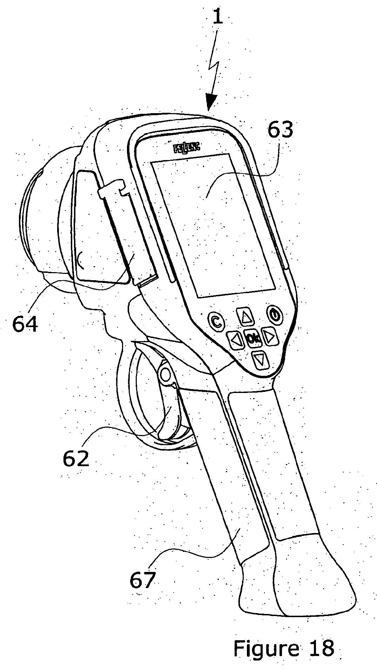

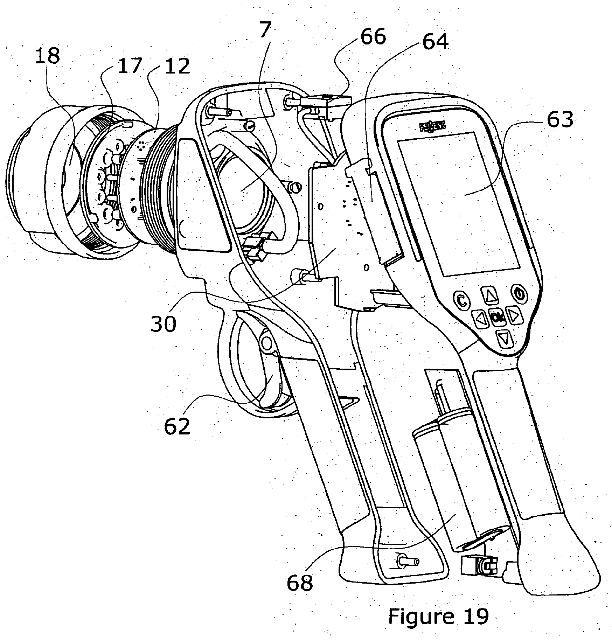

Le spectromètre optique 1 est avantageusement portable/portatif en ce que tous ses composants peuvent être réalisés suffisamment petits. L'autonomie électrique est assurée par une batterie 68 intégrée à l'appareil. Comme illustré aux figures 18 et 19, un exemple de réalisation est figuré qui peut être porté au moyen d'une poignée 67 et opéré d'une seule main par un opérateur, qui peut ainsi utiliser sa main libre pour présenter l'échantillon 4 dans la zone cible 3.

L'invention concerne encore un procédé d'utilisation d'un tel spectromètre optique 1. Afin d'obtenir des mesures, il est procédé à des acquisitions. Une première mesure est réalisée, au moyen des capteurs optiques 5, en présence d'un échantillon 4 dont on souhaite déterminer les teneurs en composés, retenus pour ledit échantillon, ce dernier étant placé dans la zone cible 3. Pendant la mesure, l'échantillon 4 est éclairé par le dispositif d'éclairage principal 12, ce dernier étant seul allumé, à l'exclusion du dispositif d'éclairage de référence 50, du troisième dispositif d'éclairage 51 et du quatrième dispositif d'éclairage 53, s'ils sont présents. Chacun des capteurs optiques 5 réalise, sensiblement en même temps, une mesure $m_i$, où l'indice i décrit l'ensemble des capteurs optiques 5, comprenant n éléments. Cette mesure est nommée "réel".

Afin d'améliorer la qualité de la mesure, il est encore procédé à une mesure par les mêmes capteurs optiques 5, tous les dispositifs d'éclairage étant éteints : dispositif d'éclairage principal 12, dispositif d'éclairage de référence 50, troisième dispositif d'éclairage 51 et quatrième dispositif d'éclairage 53. La présence de l'échantillon 4 est ici avantageuse afin de pouvoir comparer les différentes mesures. Aussi ces différentes mesures sont elles, de préférence, réalisées dans un intervalle de temps court, leur ordre relatif étant indifférent. Chacun des capteurs optiques 5 acquiert, sensiblement en même temps, une mesure $b_i$, où l'indice i décrit l'ensemble des capteurs optique 5, comprenant n éléments. Cette mesure est nommée "noir". Elle est indicative du bruit de fond optique des capteurs et de l'environnement, et est ensuite ôtée des autres mesures.

Afin d'améliorer encore la qualité de la mesure, en s'affranchissant de l'évolution de la qualité du spectre émis par le dispositif d'éclairage principal 12 dans le temps (dérive thermique en fonction de sa température propre, dérive thermique en fonction de la température ambiante, vieillissement, ...), il est utilisé un dispositif de compensation. Le dispositif d'éclairage de référence 50, déjà décrit, est conçu pour produire un spectre, qui même s'il évolue lentement avec le temps, reste à tout instant identique au spectre du dispositif d'éclairage principal 12. Ceci permet de réaliser une compensation en comparant une mesure réalisée en analysant la lumière issue du dispositif d'éclairage principal 12 après rétro diffusion par l'échantillon 4 et une mesure réalisée en analysant la lumière, théoriquement identique, directement issue de l'éclairage de référence 50. Ici aussi, les différentes mesures sont, de préférence, réalisées dans un intervalle de temps court, mais leur ordre est indifférent.

Pour obtenir cette seconde mesure, il est procédé à une mesure par les mêmes capteurs optiques 5, le dispositif d'éclairage de référence 50 étant seul allumé. Les autres dispositifs d'éclairage : dispositif d'éclairage principal 12, troisième dispositif d'éclairage 51 et quatrième dispositif d'éclairage 53 sont éteints. La présence de l'échantillon 4 est ici encore nécessaire afin de produire des conditions comparables. Chacun des capteurs optiques 5 réalise, sensiblement en même temps, une mesure $r_i$, où l'indice i décrit l'ensemble des capteurs optique 5, comprenant n éléments. Cette mesure est nommée "référence". Elle est indicative du spectre de la lumière d'éclairage lorsqu'il n'est pas modifié par l'échantillon 4. Cette mesure $r_i$ peut encore être améliorée en lui ôtant la mesure $b_i$ "noir".

A partir de ces trois mesures, il est déterminé une valeur $X_i$ corrigée, pour i compris entre 1 et n, en ôtant la mesure $b_i$ "noir" de chacune des deux autres mesures $m_i$ et $r_i$, et en comparant les deux par un quotient rapportant la mesure "réel" à la mesure "référence". Ceci peut s'exprimer par la formule $X_i = \dfrac{m_i - b_i}{r_i - b_i}$.

La compensation des différentes dérives de l'éclairage par utilisation d'un dispositif d'éclairage de référence 50 et comparaison des mesures tel que décrit permet avantageusement de réaliser une compensation de manière simple, y compris dans les conditions inévitablement variables rencontrées sur le terrain. Au contraire, un équipement de laboratoire utilise une source de lumière calibrée parfaitement connue et reproductible, et/ou une enceinte régulée thermiquement de manière précise afin de supprimer toute cause de dérive. De tels moyens ne sont bien entendus pas transposables à un spectromètre optique portatif de terrain tel que celui de l'invention.

Le procédé comprend ensuite une étape de dérivation seconde dans laquelle sont calculés des $D_i$, pour i compris entre 1 et n. Cette étape applique les formules :

$$D_1 = 2(X_1 - X_2),$$

$D_i = 2X_i - X_{i-1} - X_{i+1}$ pour i compris entre 2 et n-1, $D_n = 2(X_n - X_{n-1})$.

Dans une étape suivante, le procédé comprend encore une étape de normalisation par une norme égale à la somme des carrés $\sum_{i=1}(D_i)^2$, afin d'obtenir des $N_i$, selon la formule : $N_i = \dfrac{D_i}{\sqrt{\sum_{i=1}^{n}(D_i)^2}}$, pour i compris entre 1 et n.

Dans une étape suivante, le procédé comprend encore une étape où les $N_i$ issus des mesures sont ensuite transformés par combinaison linéaire, afin de déterminer les teneurs $M_j$ en composé de l'échantillon 4 selon la formule:

$$M_j = SM_{jn+1} + \sum_{i=1}^{n} N_i . SM_{ji},$$

où :

$N_i$ est le nombre précédemment déterminé à partir des n mesures $X_i$ issues des capteurs optiques, i compris entre 1 et n, n étant le nombre de capteurs optiques 5,

$M_j$ est la teneur du j$^{ème}$ composé, j compris entre 1 et p, p étant le nombre de composés,

$SM_{ji}$ est un coefficient caractéristique.

Les SMji sont un ensemble de coefficients caractéristiques des p composés dont l'on souhaite mesurer les teneurs et des n capteurs optiques 5 ou ce qui est équivalent, des n longueurs d'ondes associées à ces capteurs optiques. La matrice SM de dimension [n+1, p] des coefficients $SM_{ji}$ est ainsi caractéristique du couplage entre ces longueurs d'ondes et lesdits composés. La matrice SM est ainsi une caractéristique de la configuration d'un spectromètre 1 selon l'invention et de son procédé d'utilisation. Elle est stockée dans la mémoire de l'unité logique de traitement 60. Le changement de destination du spectromètre pour le rendre apte à déterminer les teneurs d'autres composés nécessite une reconfiguration comprenant la modification des longueurs d'ondes (le cas échéant de leur nombre) qui peut être réalisée par modification des filtres optiques 26, ainsi que la modification concomitante de la matrice SM, qui peut s'effectuer par modification dans la mémoire de l'unité logique de traitement 60. Une méthode de détermination de cette matrice SM caractéristique va maintenant être décrite.

La matrice de coefficients $SM_{ji}$ établit une relation linéaire entre les teneurs $M_j$ des composés, j décrivant l'ensemble des p composés et les déterminations $N_i$, i décrivant l'ensemble des n capteurs optiques 5, calculées à partir des mesures réalisées par les n capteurs optiques 5. La relation est de la forme $M_j = SM_{jn+1} + \sum_{i=1}^{n} N_i \cdot SM_{ji}$,

soit une relation linéaire affine, où $SM_{ji}$, pour i compris entre 1 et n est un poids affecté au $i^{\text{ème}}$ capteur optique 5, pour la détermination de la teneur du $j^{\text{ème}}$ composé, et $SM_{jn+1}$ est un terme supplémentaire d'offset.

Les coefficients $SM_{ji}$ de cette relation sont identifiés par toute méthode connue, à partir d'une série de mesures sur des échantillons 4, réalisée avec le spectromètre selon l'invention pour déterminer les $N_i$, les teneurs $M_j$ de ces mêmes échantillons étant connues, par exemple au moyen d'une mesure réalisée à l'aide d'un spectromètre de laboratoire. Une méthode applicable connue est la régression linéaire multiple.

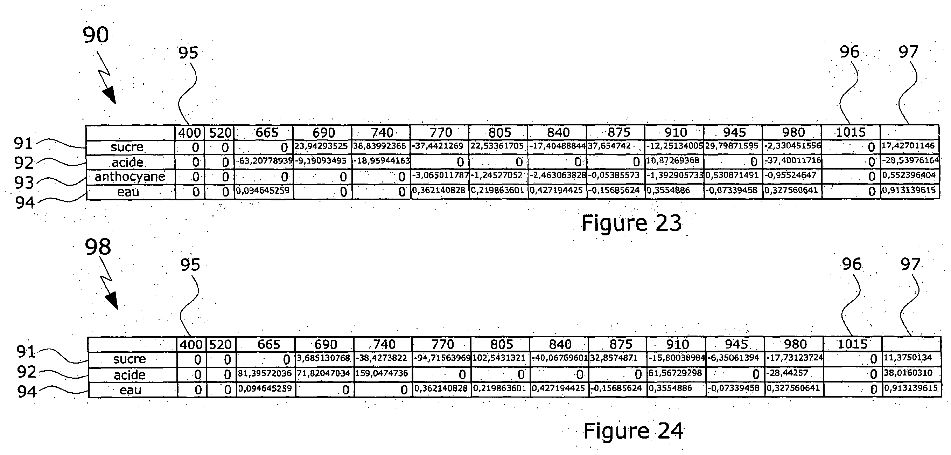

Selon un mode de réalisation illustratif particulier, appliqué au raisin, on souhaite déterminer les teneurs des quatre composés suivants : eau, sucre, polyphénols anthocyanes et acide. Pour ces composés, et pour les longueurs associés retenues, à savoir n=13 longueurs d'onde (440, 520, 665, 690, 740, 770, 805, 840, 875, 910, 945, 980, 1015), deux exemples de matrice SM sont indiqués aux figures 23 et 24. La matrice SM (90) de la figure 23 est applicable au raisin noir/rouge, et la matrice SM (98) de la figure 24 est applicable au raisin blanc. La première de ces matrices 90 comprend quatre lignes, 91-94, correspondant respectivement aux 4 composés sucre, acide, anthocyane et eau. La seconde desdites matrices 98 ne comporte que trois lignes, le composé anthocyane n'existant pour le raisin blanc. Elle comprend encore 14 colonnes. Les 13 premières colonnes correspondant aux 13 capteurs optiques, ainsi la première colonne 95 et la dernière colonne 96. Une colonne supplémentaire 97 contient les coefficients $SM_{jn+1}$ d'offset.

Le procédé comprend encore, de manière optionnelle, dans la même séquence de mesure, afin de profiter de la présence de l'échantillon 4 dans la zone cible 3, une mesure de la couleur de l'échantillon 4. Pour cela une acquisition est réalisée au moyen du capteur optique de couleur 52, le troisième dispositif d'éclairage 51 étant seul allumé afin d'éclairer l'échantillon 4. Ceci permet d'obtenir une mesure "réel couleur" $m_c$ de la couleur de l'échantillon 4.

A l'instar des autres mesures réalisées par les capteurs optiques 5 dédiés à la détermination des teneurs en composés, cette mesure peut être corrigée par soustraction d'une mesure "noir" et par compensation au moyen d'une mesure "référence".

Aussi, le procédé comprend encore une étape d'obtention d'une mesure "référence couleur" $r_c$, par acquisition au moyen du capteur optique de couleur 52, le dispositif d'éclairage de référence 50 étant seul allumé. Ainsi le capteur optique de couleur 52 est directement éclairé sans modification occasionnée par la rétro diffusion par l'échantillon 4.

De même, une mesure "noir couleur" $b_c$, est réalisée par acquisition au moyen du capteur optique de couleur 52, tous les dispositifs d'éclairage 12, 50, 51, 53 étant éteints.

Dans une autre étape, on procède ensuite à la détermination d'une valeur de couleur corrigée $X_c$, selon la

formule $X_c = \dfrac{m_c - b_c}{r_c - b_c}$.

Le procédé comprend encore, de manière optionnelle, dans la même séquence de mesure, afin de profiter de la présence de l'échantillon 4 dans la zone cible 3, une mesure par fluorescence de l'échantillon 4. Pour cela, une acquisition est réalisée au moyen du capteur optique spécifique, le quatrième dispositif d'éclairage 53, étant seul allumé afin d'éclairer l'échantillon 4 par une lumière comprenant au moins une première longueur d'onde particulière. Ceci permet d'obtenir une mesure "réel fluorescence" $m_f$ de l'échantillon 4 contrôlé.

A l'instar des autres mesures réalisées par les capteurs optiques 5, 52, cette mesure peut être corrigée par soustraction d'une mesure "noir". La compensation au moyen d'une mesure "référence" n'est pas applicable.

Aussi, le procédé comprend encore une étape d'obtention d'une mesure "noir fluorescence" $b_f$, réalisée par une acquisition au moyen du capteur optique spécifique, tous les dispositifs d'éclairage 12, 50, 51, 53 étant éteints.

Dans une autre étape, on procède ensuite à la détermination d'une valeur de fluorescence corrigée $X_f$, selon la formule $X_f = m_f - b_f$.

Afin de s'affranchir d'une éventuelle anomalie localisée sur une unique acquisition, il est avantageux de remplacer une acquisition unique par une acquisition multiple suivie d'un moyennage de ces acquisitions. Ainsi la mesure "réel" $m_i$, respectivement "réel couleur" $m_c$, respectivement "réel fluorescence" $m_f$, respectivement "référence" $r_i$, respectivement "référence couleur" $r_c$, respectivement "noir" $b_i$, respectivement "noir couleur" $b_c$, respectivement "noir fluorescence" $b_f$, est avantageusement obtenue en réalisant, une pluralité de q acquisitions, en réalisant une moyenne de ces q acquisitions, ladite moyenne étant ensuite utilisée comme mesure.

Le nombre q d'acquisitions doit être assez important pour éviter les anomalies, tout en restant suffisamment faible pour ne pas allonger inconsidérément la durée de la séquence de mesure. Compte tenu des fréquences des dispositifs d'acquisition classiques, un nombre de répétitions q = 100 est convenable.

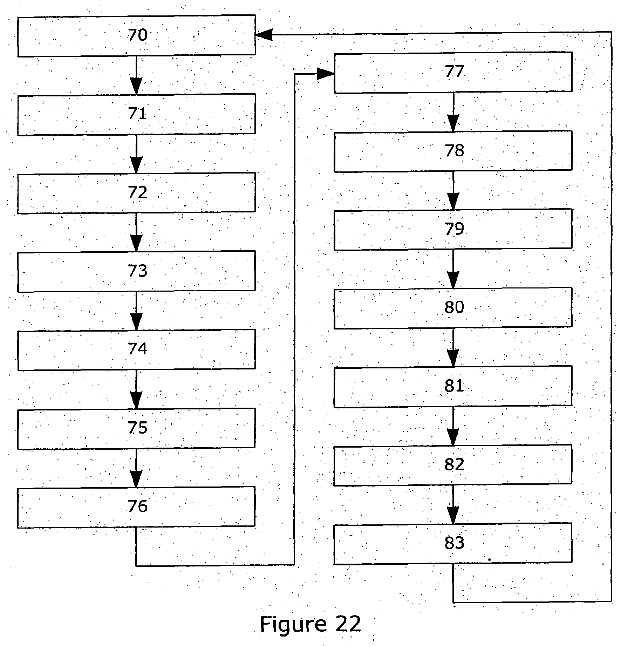

En référence à la figure 22, est décrit un déroulement du procédé d'utilisation. Une séquence typique de mesure est, par exemple, déclenchée par action sur le moyen d'entrée 62 de type gâchette, de l'interface homme machine 61. Il est procédé dans un ordre quelconque aux différentes opérations d'acquisition, accompagnées des commandes de dispositifs d'éclairage correspondants. Il peut ensuite être procédé aux différents traitements afin de produire les déterminations finales : p teneurs en composé, et le cas échéant couleur et présence/taux du composé détecté par fluorescence. Ces valeurs peuvent être affichées sur le moyen de sortie 63 de l'interface homme machine 61.

Un exemple illustratif de séquence de mesure (figure 22) comprend la suite d'opérations :

- 70 appui sur la gâchette,
- 71 mesure « noir » : $n_i$, i compris entre 1 et n, $n_c$ et $n_f$,
- 72 allumage du dispositif d'éclairage de référence 50,
- 73 mesure « référence » : $r_i$, i compris entre 1 et n, et $r_c$,
- 74 extinction du dispositif d'éclairage de référence 50,
- 75 allumage du dispositif d'éclairage principal 12,
- 76 attente 200 ms,
- 77 mesure « réel » : $m_i$, i compris entre 1 et n, $m_c$, et $m_f$,
- 78 extinction du dispositif d'éclairage principal 12,
- 79 calculs des $X_i$, i compris entre 1 et n, $X_c$, $X_f$,
- 80 stockage en mémoire,
- 81 calculs des $D_i$ et $N_i$, i compris entre 1 et n, et des $M_j$, j compris entre 1 et p,
- 82 affichage des résultats,
- 83 attente appui gâchette.

La présence d'une unité logique de traitement 60 est avantageuse en ce qu'elle permet de réaliser divers traitements sur les mesures obtenues. D'autre déterminations peuvent être calculées et le cas échéant affichées sur le moyen de sortie 63.

Ainsi en plus des mesures et déterminations instantanées déjà mentionnées, il est possible de déterminer une moyenne dans le temps des mesures et déterminations instantanées obtenues pour différents échantillons 4 successivement analysés.

Lors d'une séquence de mesure, il est encore possible de déterminer un indice de confiance par exemple basé sur l'écart type d'une mesure. L'affichage de cet indice de confiance, à l'attention de l'opérateur, lui permet de déterminer s'il doit poursuivre ou s'il peut écourter sa campagne de mesure.

Il peut encore être réalisé un test passe/passe pas d'acceptation d'une séquence de mesure. Pour un type de produit à analyser, et une configuration donnée du spectromètre 1 (longueurs d'ondes, jeu de composés) il est connu des valeurs extrêmes possibles des différentes mesures et déterminations. Un échantillon ou une séquence de mesure qui produirait une ou plusieurs mesures hors de ces valeurs extrêmes conduirait à un rejet de la séquence. Ceci permet d'éviter des séquences faussées par un échantillon 4 non adapté, ou mal positionné dans la zone cible 3, ou une anomalie optique. Un exemple d'anomalie optique est une mesure qui serait réalisée en pointant le spectromètre 1 vers une source lumineuse intense, tel le soleil. Une telle mesure serait complètement faussée par un aveuglement des capteurs optiques très sensibles.

Le procédé peut encore comprendre, dès les mesures et déterminations terminées, une étape de stockage. Ce stockage peut être local sur un moyen de stockage 64 interne et résident au spectromètre et/ou sur un moyen amovible de type carte mémoire. Il est encore possible de réaliser une étape alternative ou complémentaire de transmission vers une autre unité logique de traitement, soit en temps réel dès la mesure ou détermination disponible ou en temps différé par lots.

La séquence de mesure peut encore inclure la détermination d'une position au moyen du dispositif de géo localisation 66. Cette position peut avantageusement être jointe à une mesure ou détermination réalisée à cette position.

## Revendications

1. Spectromètre optique, en particulier du type autonome et portatif, destiné à analyser un spectre lumineux rétro diffusé par un échantillon (4) éclairé, afin d'en déduire une teneur d'au moins un composé constituant l'échantillon (4), ledit spectromètre étant organisé autour d'un axe optique (2) et comprenant :

   - une zone cible (3) centrée sur ledit axe optique (2) et apte à accueillir ledit échantillon (4),
   - une pluralité de capteurs optiques (5) visant la zone cible (3),

- un dispositif d'éclairage principal (12), apte à éclairer ledit échantillon (4), comprenant encore :

  - une chambre de mesure (7) opaque à la lumière et comprenant :
  - une ouverture (8) centrée sur ledit axe optique (2),
  - un fond intérieur (10) centré sur ledit axe optique (2) et apte à accueillir ladite pluralité de capteurs optiques (5),

**caractérisé en ce que** la chambre comprend au moins un filtre diffuseur (9) obstruant ladite ouverture (9), et **en ce que** :

  - un angle d'ouverture de la chambre de mesure (7), depuis l'ouverture (8) est inférieur ou égal à un angle de diffusion du filtre diffuseur (9) et le dispositif d'éclairage principal (12) est disposé, relativement au plan (13) perpendiculaire à l'axe optique (2) passant par le filtre diffuseur (9), du côté opposé à la zone cible (3).

2. Spectromètre optique selon la revendication 1, où le dispositif d'éclairage principal (12) est disposé de manière annulaire autour de l'axe optique (2) et est recouvert d'une protection (18) translucide et optiquement neutre.

3. Spectromètre optique selon l'une des revendications 1 ou 2, où le fond intérieur (10) et la paroi latérale interne (19) de la chambre de mesure (7) sont optiquement absorbantes.

4. Spectromètre optique selon l'une quelconque des revendications 1 à 3, où le fond intérieur (10) présente une forme sphérique centrée sur la zone cible (3) ou une forme plane perpendiculaire à l'axe optique (2).

5. Spectromètre optique selon l'une quelconque des revendications 1 à 4, où la chambre de mesure (7) présente un gain photométrique G au moins égal à 4, ledit gain photométrique G étant défini par la formule :

$$G = 100.\left(\frac{\Phi}{L}\right)^2 ,$$

avec

  Φ : diamètre de l'ouverture (8), et
  L : distance entre l'ouverture (8) et le fond intérieur (10)

6. Spectromètre optique selon l'une quelconque des revendications 1 à 5, où chacun des capteurs optiques (5) de ladite pluralité est apte à mesurer une intensité lumineuse selon une longueur d'onde donnée et comprend une photodiode (25), un filtre optique (26) disposé devant une surface sensible de ladite photodiode (26), ladite photodiode (25) étant une photodiode à grande surface réceptrice, le filtre optique (26) étant un filtre passe bande centré sur ladite longueur d'onde donnée et un amplificateur/filtre (40) présentant un gain réglé de manière à maximiser l'excursion de la mesure d'intensité lumineuse.

7. Spectromètre optique selon l'une quelconque des revendications 1 à 6, comprenant encore un dispositif d'éclairage de référence (50), reproduisant les caractéristiques lumineuses du dispositif d'éclairage principal (12), disposé à l'extérieur de la chambre de mesure (7) de manière à éclairer directement le fond intérieur (10) au travers du filtre diffuseur (9).

8. Spectromètre optique selon l'une quelconque des revendications 1 à 7, comprenant encore un système de mesure de la couleur (51, 52) de l'échantillon (4) comprenant un troisième dispositif d'éclairage (51) et un capteur optique de couleur (52), tous deux visant la zone cible (3) et étant disposés sur le fond intérieur (10), sensiblement sur l'axe optique (2).

9. Spectromètre optique selon l'une quelconque des revendications 1 à 8, comprenant encore un système de mesure par fluorescence de l'échantillon (4) comprenant un quatrième dispositif d'éclairage (53) émettant selon une longueur d'onde centrale de 380nm, et un capteur optique spécifique tous deux visant la zone cible (3) et étant disposés sur le fond intérieur (10), sensiblement sur l'axe optique (2), le capteur optique spécifique présentant une longueur d'onde centrale de 522nm, afin de permettre une détection du Botrytis.

**10.** Spectromètre optique selon l'une quelconque des revendications 1 à 9, comprenant encore un dispositif de géo localisation (66)

**11.** Procédé d'utilisation d'un spectromètre optique selon l'une quelconque des revendications précédentes comprenant encore un dispositif d'éclairage de référence (50), reproduisant les caractéristiques lumineuses du dispositif d'éclairage principal (12), disposé à l'extérieur de la chambre de mesure (7) de manière à éclairer directement le fond intérieur (10) au travers du filtre diffuseur (9),
le procédé comprenant les étapes suivantes:

- obtention par acquisition, d'une mesure "réel" $m_1$, où l'indice i décrit de 1 à n la pluralité de capteurs optiques (5), en présence d'un échantillon (4) dans la zone cible (3), le dispositif d'éclairage principal (12) étant seul allumé afin d'éclairer ledit échantillon (4),
- obtention par acquisition, d'une mesure "noir" $b_1$, où l'indice i décrit de 1 à n la pluralité de capteurs optiques (5), tous les dispositifs d'éclairage (12, 50, 51, 53) étant éteints, comprenant encore les étapes suivantes
- obtention par acquisition, d'une mesure "référence" $r_i$, où l'indice i décrit de 1 à n la pluralité de capteurs optiques (5), le dispositif d'éclairage de référence (50) étant seul allumé afin d'éclairer directement lesdits capteurs optiques (5) .

**12.** Procédé selon la revendication 11, comprenant encore une étape de détermination de valeur $X_1$ corrigée pour i compris entre 1 et n, telle que $X_i = \dfrac{m_i - b_i}{r_i - b_i}$, une étape de dérivation seconde, afin d'obtenir $D_1$ pour i compris entre 1 et n, telle que $D_1 = 2(X_1 - X_2)$, $D_i = 2X_i - X_{i-1} -, X_{i+1}$ pour i compris entre 2 et n-1, $D_n, = 2(X_n - X_{n-1})$, une étape de normalisation, afin d'obtenir $N_1$, pour i compris entre 1 et n, telle que $N_i = \dfrac{D_i}{\sqrt{\sum_{i=1}^{n}(D_i)^2}}$ et une étape de détermination de la teneur en composé $M_j$ de l'échantillon (4), où l'indice j décrit de 1 à p l'ensemble des composés dont on mesure les teneurs, par application de la formule :

$$M_j = SM_{jn+1} + \sum_{i=1}^{n} N_i . SM_{ji}$$

où $SM_{j1}$ est un coefficient caractéristique.

**13.** Procédé selon l'une quelconque des revendications 11 ou 12, d'utilisation d'un spectromètre optique comprenant encore un système de mesure de la couleur (51, 52) de l'échantillon (4) comprenant un troisième dispositif d'éclairage (51) et un capteur optique de couleur (52), tous deux visant la zone cible (3) et étant disposés sur le fond intérieur (10), sensiblement sur l'axe optique (2),
le procédé , comprenant encore une étape de mesure "réel couleur" $m_c$ de la couleur de l'échantillon (4), au moyen du capteur optique de couleur (52), le troisième dispositif d'éclairage (51) étant seul allumé afin d'éclairer l'échantillon (4), une étape d'obtention par acquisition au moyen du capteur optique de couleur (52), d'une mesure "référence couleur" $r_c$, le dispositif d'éclairage de référence (50) étant seul allumé afin d'éclairer directement ledit capteur optique de couleur (52), une étape d'obtention par acquisition au moyen du capteur optique de couleur (52), d'une mesure "noir couleur" $b_c$, tous les dispositifs d'éclairage (12, 50, 51, 53) étant éteints et une étape de détermination de valeur de couleur corrigée $X_c$, telle que

$$X_c = \dfrac{m_c - b_c}{r_c - b_c}.$$

**14.** Procédé selon l'une quelconque des revendications 11 à 13, comprenant encore une étape de mesure "réel fluorescence" $m_f$ de l'échantillon (4), au moyen du capteur optique spécifique le quatrième dispositif d'éclairage (53) étant seul allumé afin d'éclairer l'échantillon (4), une étape d'obtention par acquisition au moyen du capteur optique spécifique d'une mesure "noir fluorescence" $b_f$, tous les dispositifs d'éclairage (12, 50, 51, 53) étant éteints et une étape de détermination de valeur de fluorescence corrigée $X_f$, telle que $X_f = m_f - b_f$.

**Patentansprüche**

1. Optisches Spektrometer, insbesondere vom autonomen und tragbaren Typ, das zum Analysieren eines Lichtspektrums bestimmt ist, das von einer befeuchteten Probe (4) zurückgestrahlt wird, um daraus den Gehalt von mindestens einer Verbindung abzuleiten, welche die Probe (4) bildet, wobei das Spektrometer um eine optische Achse (2) ausgerichtet ist und Folgendes umfasst:

   - einen Zielbereich (3), der auf der optischen Achse (2) zentriert und dazu geeignet ist, die Probe (4) aufzunehmen,
   - eine Vielzahl von optischen Sensoren (5), die auf den Zielbereich (3) gerichtet sind,
   - eine Hauptbeleuchtungsvorrichtung (12), die geeignet ist, um die Probe (4) zu beleuchten,

   weiterhin umfassend:

   - eine Messkammer (7), die für das Licht undurchlässig ist und umfassend:
   - eine Öffnung (8), die auf der optischen Achse (2) zentriert ist,
   - einen Innenboden (10), der auf der optischen Achse (2) zentriert und geeignet ist, um die Vielzahl von optischen Sensoren (5) aufzunehmen,

   **dadurch gekennzeichnet, dass** die Kammer mindestens einen Diffusorfilter (9) umfasst, der die Öffnung (9) blockiert, und dadurch, dass:

   - ein Öffnungswinkel der Messkammer (7) von der Öffnung (8) kleiner gleich einem Diffusionswinkel des Diffusorfilters (9) ist und die Hauptbeleuchtungsvorrichtung (12) in Bezug auf die Ebene (13) senkrecht zu der optischen Achse (2), die durch den Diffusorfilter (9) hindurchgeht, gegenüber dem Zieibereich (3) angeordnet ist.

2. Optisches Spektrometer nach Anspruch 1, wobei die Hauptbeleuchtungsvorrichtung (12) ringförmig um die optische Achse (2) angeordnet und mit einem lichtundurchlässigen und optisch neutralen Schutz (18) bedeckt ist.

3. Optisches Spektrometer nach einem der Ansprüche 1 oder 2, wobei der Innenboden (10) und die innere Seitenwand (19) der Messkammer (7) optisch absorbierend sind.

4. Optisches Spektrometer nach einem der Ansprüche 1 bis 3, wobei der Innenboden (10) eine kugelförmige Form, die auf den Zielbereich (3) zentriert ist oder eine ebene Form darstellt, die zu der optischen Achse (2) senkrecht verläuft.

5. Optisches Spektrometer nach einem der Ansprüche 1 bis 4, wobei die Messkammer (7) eine photometrische Verstärkung G von mindestens 4 aufweist, wobei die photometrische Verstärkung G durch die folgende Formel definiert ist:

$$G = 100 \left( \frac{\Phi}{L} \right)^2 ,$$

   wobei

   $\Phi$: Durchmesser der Öffnung (8) und
   L: Abstand zwischen der Öffnung (8) und dem Innenboden (10).

6. Optisches Spektrometer nach einem der Ansprüche 1 bis 5, wobei jeder der optischen Sensoren (5) der Vielzahl geeignet ist, um eine Lichtintensität gemäß einer gegebenen Wellenlänge zu messen und eine Photodiode (25) umfasst, wobei ein optischer Filter (26) vor einer empfindlichen Oberfläche der Photodiode (26) angeordnet ist, wobei die Photodiode (25) eine Photodiode mit einer großen Aufnahmefläche ist, wobei der optische Filter (26) ein Bandpassfilter ist, der auf die gegebene Wellenlänge zentriert ist und wobei ein Verstärker/Filter (40) eine geregelte Verstärkung aufweist, um die Messabweichung der Lichtintensität zu maximieren.

7. Optisches Spektrometer nach einem der Ansprüche 1 bis 6, weiterhin umfassend eine Referenzbeleuchtungsvorrichtung (50), welche die Leuchteigenschaften der Hauptbeleuchtungsvorrichtung (12) reproduziert und außerhalb der Messkammer (7) angeordnet ist, um den Innenboden (10) durch den Diffusorfilter (9) direkt zu beleuchten.

8. Optisches Spektrometer nach einem der Ansprüche 1 bis 7, weiterhin umfassend ein Farbmesssystem (51, 52) für die Probe (4), umfassend eine dritte Beleuchtungsvorrichtung (51) und einen optischen Farbsensor (52), die beide auf den Zielbereich (3) gerichtet und auf dem Innenboden (10) etwa auf der optischen Achse (2) angeordnet sind.

9. Optisches Spektrometer nach einem der Ansprüche 1 bis 8, weiterhin umfassend ein Fluoreszenzmesssystem für die Probe (4), umfassend eine vierte Beleuchtungsvorrichtung (53), die mit einer zentralen Wellenlänge von 380 nm emittiert und einen spezifischen optischen Sensor, die beide auf den Zielbereich (3) gerichtet und auf dem Innenboden (10) etwa auf der optischen Achse (2) angeordnet sind, wobei der spezifische optische Sensor eine zentrale Wellenlänge von 522 nm aufweist, um die Detektion von Grauschimmel zu ermöglichen.

10. Optisches Spektrometer nach einem der Ansprüche 1 bis 9, weiterhin umfassend eine Ortsbestimmungsvorrichtung (66).

11. Verfahren zur Verwendung eines optischen Spektrometers nach einem der vorhergehenden Ansprüche, weiterhin umfassend eine Referenzbeleuchtungsvorrichtung (50), welche die Leuchteigenschaften der Hauptbeleuchtungsvorrichtung (12) reproduziert und außerhalb der Messkammer (7) angeordnet ist, um den Innenboden (10) durch den Diffusorfilter (9) direkt zu beleuchten, wobei das Verfahren die folgenden Schritte umfasst:

- Erhalten einer "reellen" Messung $m_i$ durch Erfassung, wobei der Index i von 1 bis n die Vielzahl der optischen Sensoren (5) beschreibt, in Gegenwart einer Probe (4) im Zielbereich (3), wobei nur die Hauptbeleuchtungsvorrichtung (12) eingeschaltet ist, um die Probe (4) zu beleuchten,
- Erhalten einer "dunklen" Messung $b_i$ durch Erfassung, wobei der Index i von 1 bis n die Vielzahl der optischen Sensoren (5) beschreibt, wobei alle Beleuchtungsvorrichtungen (12, 50, 51, 53) abgeschaltet sind,

weiterhin umfassend die folgenden Schritte:

- Erhalten einer "Referenz"-Messung η durch Erfassung, wobei der Index i von 1 bis n die Vielzahl der optischen Sensoren (5) beschreibt, wobei nur die Referenzbeleuchtungsvorrichtung (50) eingeschaltet ist, um die optischen Sensoren (5) direkt zu beleuchten.

12. Verfahren nach Anspruch 11, weiterhin umfassend einen Bestimmungsschritt für den korrigierten Wert $x_i$ für i zwischen 1 und n, sodass

$$X_i = \frac{m_i - b_i}{r_i - b_i},$$

, einen Schritt der zweiten Ableitung, um $D_i$ für i zwischen 1 und n zu erhalten, sodass $D_1 = 2(X_1 - X_2)$, $D_i = 2X_i - X_{i-1} - X_{i+1}$ für i zwischen 2 und n-1, $D_n = 2(X_n - X_{n-1})$ einen Normalisierungsschritt,

um $N_i$ für i zwischen 1 und n zu erhalten, sodass

$$N_i = \frac{D_i}{\sqrt{\sum_{i=1}^{n}(D_i)^2}}$$

und einen Bestimmungsschritt des Verbindungsgehalts $M_j$ der Probe (4), wobei der Index j von 1 bis p die Gesamtheit der Verbindungen beschreibt, deren Gehalt gemessen wird, unter Anwendung der folgenden Formel:

$$M_j = SM_{jn+1} + \sum_{i=1}^{n} N_i . SM_{ji}$$

wobei $SM_{ji}$ ein charakteristischer Koeffizient ist.

**13.** Verfahren nach einem der Ansprüche 11 oder 12 zur Verwendung eines optischen Spektrometers, weiterhin umfassend ein Farbmesssystem (51, 52) für die Probe (4), umfassend eine dritte Beleuchtungsvorrichtung (51) und einen optischen Farbsensor (52), die beide auf den Zielbereich (3) gerichtet und auf dem Innenboden (10) etwa auf der optischen Achse (2) angeordnet sind,

wobei das Verfahren weiterhin Folgendes umfasst: einen Schritt zum Messen der "reellen Farbe" $m_c$ der Farbe der Probe (4), mithilfe des optischen Farbsensors (52), wobei nur die dritte Beleuchtungsvorrichtung (51) eingeschaltet ist, um die Probe (4) zu beleuchten, einen Schritt zum Erhalten einer Messung der "Referenzfarbe" $r_c$ durch Erfassung mithilfe des optischen Farbsensors (52), wobei nur die Referenzbeleuchtungsvorrichtung (50) eingeschaltet ist, um den optischen Farbsensor (52) direkt zu beleuchten, einen Schritt zum Erhalten einer Messung der "dunklen Farbe" $b_c$ durch Erfassung mithilfe des optischen Farbsensors (52), wobei alle Beleuchtungsvorrichtungen (12, 50, 51, 53) abgeschaltet sind und einen Bestimmungsschritt für den korrigierten Farbwert $x_c$, sodass $$X_c = \frac{m_c - b_c}{r_c - b_c}.$$

**14.** Verfahren nach einem der Ansprüche 11 bis 13, weiterhin umfassend einen Schritt zum Messen der "reellen Fluoreszenz" $m_f$ der Probe (4) mithilfe des spezifischen optischen Sensors, wobei nur die vierte Beleuchtungsvorrichtung (53) eingeschaltet ist, um die Probe (4) zu beleuchten, einen Schritt zum Erhalten einer Messung der "dunklen Fluoreszenz" $b_f$ durch Erfassung mithilfe des spezifischen optischen Sensors, wobei alle Beleuchtungsvorrichtungen (12, 50, 51, 53) abgeschaltet sind und einen Bestimmungsschritt für den korrigierten Fluoreszenzwert $x_f$, sodass $X_f = m_f - b_f$.

**Claims**

**1.** An optical spectrometer, in particular of the stand-alone and portable type, intended for analysing a retro light spectrum diffused by an illuminated sample (4), in order to deduce from it a content of at least one compound constituting the sample (4), said spectrometer being arranged around an optical axis (2) and comprising:

- a target area (3) centred on said optical axis (2) and capable of receiving said sample (4);
- a plurality of optical sensors (5)
aiming at the target area (3);
- a principal illuminating device (12), capable of illuminating said sample (4),

further comprising:

- a measuring chamber (7) opaque to light and comprising:
- an opening (8) centred on said optical axis (2);
- an interior base (10) centred on said optical axis (2) and capable of receiving said plurality of optical sensors (5);

**characterised in that** the chamber consists of at least one diffusing filter (9) covering said opening (9), **in that**:

- an angle of opening of the measuring chamber (7) from the opening (8) is less than or equal to an angle of diffusion of the diffusing filter (9)

and
the principal illuminating device (12) is disposed relative to the plane (13), perpendicular to the optical axis (2) passing by the diffusing filter (9), on the side opposite the target area (3).

**2.** The optical spectrometer in accordance with Claim 1, where the principal illuminating device (12) is disposed, in a circular manner, around the optical axis (2) and is covered by a translucent protective layer (18) and is optically neutral.

**3.** The optical spectrometer in accordance with one of Claims 1 or 2, wherein the interior base (10) and the internal side panel (19) of the measuring chamber (7) are optically absorbent.

**4.** The optical spectrometer in accordance with any one of Claims 1 to 3, wherein the interior base (10) presents a spherical form centred on the target area (3) or a planar form perpendicular to the optical axis (2).

5. The optical spectrometer in accordance with any one of Claims 1 to 4, wherein the measuring chamber (7) shows a photometric gain G at least equal to 4, said photometric gain G being defined by the formula:

$$G = 100 \left( \frac{\Phi}{L} \right)^2$$

with

Φ: diameter of the opening (8), and
L: distance between the opening (8) and the interior base (10).

6. The optical spectrometer in accordance with any one of Claims 1 to 5, wherein each of the optical sensors (5) of said plurality is capable of measuring a luminous intensity in accordance with a given wavelength and comprises a photodiode (25) and an optical filter (26) disposed in front of a sensitive surface of said photodiode (25), said photodiode (25) being a photodiode with a large receiving surface, the optical filter (26) being a band-pass filter centred on said given wavelength and an amplifier/filter (40) showing a gain regulated in such a manner as to maximise the swing of the measure of luminous intensity.

7. The optical spectrometer in accordance with any one of Claims 1 to 6, further comprising a reference illuminating device (50), reproducing the luminous features of the principal illuminating device (12), disposed outside the measuring chamber (7) in such a way as to directly light up the interior base (10) through the diffusing filter (9).

8. The optical spectrometer in accordance with any one of Claims 1 to 7, further comprising a colour measuring system (51, 52) of the sample (4) comprising a third illuminating device (51) and an optical colour sensor (52), both aiming at the target area (3) and being disposed on the interior base (10), mainly on the optical axis (2).

9. The optical spectrometer in accordance with any one of Claims 1 to 8, further comprising a system of measuring by fluorescence of the sample (4) comprising a fourth illuminating device (53) emitting light in accordance with a central wavelength of 380 nm, and a specific optical sensor, both aiming at the target area (3) and being disposed on the interior base (10), mainly on the optical axis (2), the specific optical sensor showing a central wavelength of 522 nm, in order to enable the detection of botrytis.

10. The optical spectrometer in accordance with any one of Claims 1 to 9, further comprising a geo-localisation device (66).

11. A method of utilising an optical spectrometer in accordance with any one of the preceding claims, further comprising a reference illuminating device (50), reproducing the luminous features of the principal illuminating device (12), disposed outside the measuring chamber (7) in such a way as to directly light up the interior base (10) through the diffusing filter (9), with the method comprising the following steps:

- obtaining by acquisition, a "real" measure $m_i$, wherein the index i describes from 1 to n the plurality of optical sensors (5), in the presence of a sample (4) in the target area (3), the principal illuminating device (12) only being illuminated in order to light up said sample (4);
- obtaining by acquisition, a "black" measure $b_i$, wherein the index i describes from 1 to n the plurality of optical sensors (5), all the illuminating devices (12, 50, 51, 53) being switched off, further comprising the following steps:

    - obtaining by acquisition a "reference" measure $r_i$, where the index i describes from 1 to n the plurality of optical sensors (5), the reference illuminating device (50) only being lit up in order to directly light up said optical sensors (5).

12. The method in accordance with Claim 11, further comprising a stage of determining a value $X_i$, adjusted for i

comprising a value between 1 and n, such that $X_i = \dfrac{m_i - b_i}{r_i - b_i}$, a second derivation step, in order to obtain

$D_i$ for i comprising a value between 1 and n, such that $D_1 = 2(X_1 - X_2), D_i = 2X_i - X_{i-1} - X_{i+1}$ for i comprising a value between 2 and n-1, $D_n = 2(X_n - X_{n+1})$, a standardisation stage, in order to obtain $N_i$, for i comprising a value between 1 and n, such that $N_i = \dfrac{D_i}{\sqrt{\sum\limits_{i=1}^{n}(D_i)^2}}$ and a stage to determine the content comprising $M_j$ of the sample (4), where the index j describes from 1 to p all the compounds, of which the content is measured, by applying the formula:

$$M_j = SM_{jn+1} + \sum_{i=1}^{n} N_i . SM_{ji}$$

wherein $SM_{ji}$ is a characteristic coefficient.

**13.** The method in accordance with any one of Claims 11 or 12, of using an optical spectrometer further comprising a system (51, 52) for measuring the colour of the sample (4) comprising a third illuminating device (51) and a colour optical sensor (52), both of them aiming at the target area (3) and being disposed on the interior base (10), mainly on the optical axis (2), the method further comprising a stage of measuring "real colour" $m_c$ of the colour of the sample (4), by means of the colour optical sensor (52), the third illuminating device (51) only being lit up in order to light up the sample (4), a stage of obtaining by acquisition by means of the colour optical sensor (52), a "reference colour" measurement $r_c$, the reference illuminating device (50) only being lit up in order to directly light up said colour optical sensor (52), a stage of obtaining by acquisition, by means of the colour optical sensor (52), a "black colour" measurement $b_c$ all the illuminating devices (12, 50, 51, 53) being switched off and a stage of determining the value of adjusted colour $X_c$ such that , $X_c = \dfrac{m_c - b}{r_c - b,}$

**14.** The method in accordance with any one of Claims 11 to 13, further comprising a stage of measuring "real fluorescence" $m_f$ of the sample (4), by means of a specific optical sensors, the fourth illuminating device (53) only being lit up in order to light up the sample (4), a stage of obtaining by acquisition by means of the specific optical sensor, a measurement of "black fluorescence" $b_f$, all the illuminating devices (12, 50, 51, 53) being switched off, and a stage of determining the value of adjusted fluorescence $X_f$, such that $X_f = m_f - b_f$.

Figure 1

Figure 2

Figure 3

Figure 4

Figure 5

21

Figure 6

Figure 7

A

22

1    2 3    4    5    6    n    λ

Figure 8

Figure 9

Figure 10

Figure 11

Figure 12

Figure 13

Figure 14

56

33

31

26

30

51

53 52 25

2

Figure 15

56 33

31

30

2

Figure 16

Figure 17

Figure 18

Figure 19

Figure 20

Figure 21

Figure 22

EP 2 475 975 B1

**90**

**95** **96** **97**

| | 400 | 520 | 665 | 690 | 740 | 770 | 805 | 840 | 875 | 910 | 945 | 980 | 1015 | |
|---|---|---|---|---|---|---|---|---|---|---|---|---|---|---|
| sucre | 0 | 0 | 0 | 23,94293525 | 38,83992366 | -37,4421269 | 22,53361705 | -17,40488844 | 37,654742 | -12,25134005 | 29,79871595 | -2,330451556 | 0 | 17,42701146 |
| acide | 0 | 0 | -63,20778939 | -9,19093495 | -18,95944163 | 0 | 0 | 0 | 0 | 10,87269368 | 0 | -37,40011716 | 0 | -28,53976164 |
| anthocyane | 0 | 0 | 0 | 0 | 0 | -3,065011787 | -1,24527052 | -2,463063828 | -0,05385573 | -1,3929057330 | 0,530871491 | -0,95524647 | 0 | 0,552396404 |
| eau | 0 | 0 | 0,094645259 | 0 | 0 | 0,362140828 | 0,219863601 | 0,427194425 | -0,15685624 | 0,3554886 | -0,07339458 | 0,327560641 | 0 | 0,913139615 |

91, 92, 93, 94

**Figure 23**

**98**

**95** **96** **97**

| | 400 | 520 | 665 | 690 | 740 | 770 | 805 | 840 | 875 | 910 | 945 | 980 | 1015 | |
|---|---|---|---|---|---|---|---|---|---|---|---|---|---|---|
| sucre | 0 | 0 | 0 | 3,685130768 | -38,4273822 | -94,71563969 | 102,5431321 | -40,06769601 | 32,8574871 | -15,80038984 | -6,35061394 | -17,73123724 | 0 | 11,3750134 |
| acide | 0 | 0 | 81,39572036 | 71,82047034 | 159,0474736 | 0 | 0 | 0 | 0 | 61,56729298 | 0 | -28,44257 | 0 | 38,0160310 |
| eau | 0 | 0 | 0,094645259 | 0 | 0 | 0,362140828 | 0,219863601 | 0,427194425 | -0,15685624 | 0,3554886 | -0,07339458 | 0,327560641 | 0 | 0,913139615 |

91, 92, 94

**Figure 24**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 9838494 A
- US 2004130720 A
- US 20060118726 A